# EUROPEAN PATENT APPLICATION

(11) **EP 1 512 749 A2**
(43) Date of publication of application: **09.03.2005**
(21) Application number: 04255383.4
(22) Date of filing: 03.09.2004
(51) Int. Cl.: C12N 15/85, C12N 15/11, C12N 15/63, C12N 15/00, C12N 15/90

(54) **DNA to be introduced into biogenic gene, gene introducing vector, cell, and method for introducing information into biogenic gene**

(30) Priority: 05.09.2003 JP 2003313519
(71) Applicant: SONY CORPORATION, Tokyo 141 (JP)
(72) Inventor: Ohto, Yasunori, c/o Sony-Kihara, Shinagawa-ku Tokyo (JP)
(74) Representative: DeVile, Jonathan Mark, Dr.

(57) **Abstract**

A method and an apparatus for introducing information into a living organism reliably and in a way which is unlikely to disturb the original function of the living organism. Information to be introduced into a biogenic DNA is prepared in a step. This information is compressed in the next Step. The compressed data is converted into base sequence data in the next step. The base sequence data has an error correction code inserted thereinto in the next step, has a buffer sequence inserted thereinto in the next Step, and has a sequence for positive selection inserted thereinto in the next step. If the position of introduction is determined in the next step to be a sequence complementary to the untranslated region, then an intron sequence is added. If the position of introduction is determined in the next step to be intron, then the next step is skipped and the position for introduction (which has the least effect on the living organism) is determined in the following step immediately after the skipped step. The present invention may be applied to genetically modified foods.

## Description

### BACKGROUND OF THE INVENTION

The present invention relates to a DNA to be introduced into a biogenic gene, a gene introducing vector, a cell, a method for introducing information into a biogenic gene, an apparatus and a method for data processing, a recording medium, and a program. More particularly, the present invention relates to a DNA to be introduced into a biogenic gene, a gene introducing vector, a cell, a method for introducing information into a biogenic gene, an apparatus and a method for data processing, a recording medium, and a program, all of which permit one to introduce information into a living organism without fail or at least reliably.

It has been common practice to record details of gene manipulation, such as modification of gene and introduction of gene into a living organism, in notebooks, paper media, personal computers, etc. for its follow up.

There has been proposed to set up a site for recombination in a data molecule and introduce the data molecule into a non-coding region (or intron region) of a gene. (See patent document 1: Japanese Unexamined Patent Publication No. 2002-541539 (p. 68), for example.)

Even though any gene manipulation performed on living organisms is recorded in notebooks, paper media, personal computers, etc. for its follow up, it would be impossible to know what gene manipulation has been performed on living organisms if there is no knowledge about the relation between living organisms and follow-up information.

There may be an instance in which individual cells which have undergone different gene manipulations are cultured in a plurality of different culture dishes. In this case, the culture dishes are given serial numbers, so that each number corresponds to managing information about the gene manipulation which has been performed on the cell in each numbered culture dish, and a correspondence list is created. By inputting such a list to a computer or the like, the managing information correspond to living organisms have been managed. The disadvantage of this practice is that once the list showing such correspondence becomes unavailable due to loss or the like, it would be impossible to know what gene manipulation the cell in each culture dish has received.

One conceivable way to cope with this situation is to introduce a data molecule into an intron, thereby recording the follow-up information in a living organism itself, as disclosed in Patent Document 1. Unfortunately, there is a possibility that an intron has a certain function in a living organism. Therefore, the introduction of an data molecule into an intron might disturb the physiological function inherent to individual living organisms.

### SUMMARY OF THE INVENTION

Various aspects and features of the present invention are defined in the appended claims.

The present invention provides a DNA which is encoded with information to be introduced into a biogenic DNA of a genetically manipulated organism and which has an error-correcting base inserted thereinto.

The DNA may additionally have a buffer base inserted thereinto so that it decreases in homology to the base sequence of DNA of more than one species of organisms.

The DNA may additionally have a drug tolerant gene for positive selection.

The DNA may additionally have a drug tolerant gene for negative selection.

The DNA may additionally have the base of intron sequence which is recognized as the intron region by the genetically manipulated organism.

Embodiments of the present invention can provide a vector for gene introduction, which is characterized in containing a DNA which is encoded with information to be introduced into a biogenic DNA of a genetically manipulated organism and which has an error-correcting base inserted thereinto.

Embodiments of the present invention can provide a cell, which is characterized in possessing a DNA which is encoded with information to be introduced into a biogenic DNA of a genetically manipulated organism and which has an error-correcting base inserted thereinto.

Embodiments of the present invention can provide a method for introduction of information into a biogenic gene, the method including a step of designing the base sequence of a DNA to be introduced into a biogenic DNA, the DNA being encoded with information to be introduced into a biogenic DNA of a genetically manipulated organism and having an error-correcting base inserted thereinto, a step of synthesizing the DNA having the thus designed base sequence, and a step of introducing the thus synthesized DNA into a genetically manipulated gene.

The above-mentioned method for introduction of information into a biogenic gene may be accomplished in such a way that the DNA is introduced into a region not encoded with protein out of the base sequence of the genetically manipulated gene, when the DNA is introduced into the genetically manipulated gene.

The above-mentioned method for introduction of information into a biogenic gene is characterized in that the region not encoded with protein is an intron region.

The above-mentioned method for introduction of information into a biogenic gene is characterized in that the region not encoded with protein is a region of the base sequence complementary to the untranslated region which is not translated into the protein, of the base sequence of mRNA to which the gene has been transcribed, out of the biogenic DNA.

The above-mentioned method for introduction of information into a biogenic gene is characterized in that the base sequence of the DNA to be introduced into the biogenic DNA further contains an intron sequence which is recognized as the intron region by the genetically manipulated organism.

The above-mentioned method for introduction of information into a biogenic gene includes a step of acquiring a base sequence pattern similar to that present in more than one species of organisms according to the base sequence of DNA of more than one species of organisms before introducing the synthesized DNA into the genetically manipulated gene, a step of selecting a site of base sequence having the least similarity to the base sequence pattern from the region not encoded with protein of the genetically manipulated gene, and a step of introducing the synthesized DNA into the site selected as the site having the least similarity to the base sequence pattern at the time of introducing the DNA into the genetically manipulated gene.

The above-mentioned method for introduction of information into a biogenic gene includes a step of acquiring a base sequence pattern similar to that present in more than one species of organisms according to the base sequence of DNA of more than one species of organisms before designing the base sequence of the DNA to be introduced the biogenic DNA, and a step of inserting a buffer base sequence so that the similarity to the acquired base sequence pattern becomes least, thereby designing the the base sequence of the DNA to be introduced into the biogenic DNA at the time of designing the base sequence of the DNA to be introduced into biogenic DNA.

Embodiments of the present invention can provide a first data processing apparatus which includes means to convert binary data containing information to be introduced into a biogenic DNA of a genetically manipulated organism into base sequence data and means to insert base data for error correction into the base sequence data obtained by the converting means.

Embodiments of the present invention can provide a first data processing method which includes a step of converting binary data containing information to be introduced into a biogenic DNA of a genetically manipulated organism into base sequence data and a step of inserting base data for error correction into the base sequence data obtained by the converting step.

Embodiments of the present invention can provide a first program stored in a recording medium, which includes a step of converting binary data containing information to be introduced into a biogenic DNA of a genetically manipulated organism into base sequence data and a step of inserting base data for error correction into the base sequence data obtained by the converting step.

Embodiments of the present invention can provide a first program which causes a computer to execute a step of converting binary data containing information to be introduced into a biogenic DNA of a genetically manipulated organism into base sequence data and a step of inserting base data for error correction into the base sequence data obtained by the converting step.

Embodiments of the present invention can provide a second data processing apparatus which includes means to correct errors in base sequence data according to the base sequence for error correction inserted into the base sequence data, and means to convert the base sequence data, which has been error-corrected by the correcting means, into binary data.

Embodiments of the present invention can provide a second data processing method which includes a step of correcting errors in base sequence data according to the base sequence for error correction inserted into the base sequence data, and a step of converting the base sequence data, which has been error-corrected by the correcting step, into binary data.

Embodiments of the present invention can provide a second program recorded in a recording medium, which includes a step of correcting errors in base sequence data according to the base sequence for error correction inserted into the base sequence data, and a step of converting the base sequence data, which has been error-corrected by the correcting step, into binary data.

Embodiments of the present invention can provide a second program which causes a computer to execute a step of correcting errors in base sequence data according to the base sequence for error correction inserted into the base sequence data, and a step of converting the base sequence data, which has been error-corrected by the correcting step, into binary data.

According to embodiments of the present invention, the first information processing apparatus and method, recording medium, and program are characterized in that the binary data containing information to be introduced into a biogenic DNA of a genetically manipulated organism is converted into base sequence data and base data for error correction is inserted into the converted base sequence.

According to embodiments of the present invention, the second information processing apparatus and method, recording medium, and program are characterized in that errors in base sequence data is corrected according to the base sequence for error correction inserted into the base sequence data, and the error-corrected base sequence data is converted into binary data.

The present invention may be applicable to genetic recombination.

A first aspect of the present invention permits the introduction of information into a living organism. Particularly, it permits the reliable introduction of information into a living organism without disturbance with its original functions or with at least a reduced likelihood of disturbing its original functions.

A second aspect of the present invention permits the introduction of information into a living organism. Particularly, it permits the reliable introduction of information into a living organism without disturbance with its original functions or with at least a reduced likelihood of disturbing its original functions.

A third aspect of the present invention permits the introduction of information into a living organism. Particularly, it permits the reliable introduction of information into a living organism without disturbance with its original functions or with at least a reduced likelihood of disturbing its original functions.

A fourth aspect of the present invention permits the introduction of information into a living organism. Particularly, it permits the reliable introduction of information into a living organism without disturbance with its original functions or with at least a reduced likelihood of disturbing its original functions.

A fifth aspect of the present invention permits the introduction of information into a living organism. Particularly, it permits the reliable introduction of information into a living organism without disturbance with its original functions or with at least a reduced likelihood of disturbing its original functions.

A sixth aspects of the present invention permits the acquisition of the information introduced into a living organism. Particularly, it permits the reliable acquisition of the information introduced into a living organism.

### BRIEF DESCRIPTION OF THE DRAWINGS

The invention will now be described by way of example with reference to the accompanying drawings, throughout which like parts are referred to by like references, and in which:
FIG. 1 is a diagram showing an example of the construction of the information processing system which is based on the present invention;
FIG. 2 is a block diagram showing an example of the construction of the sequence design apparatus shown in FIG. 1;
FIG. 3 is a block diagram showing an example of the construction of the information acquiring apparatus shown in FIG. 1;
FIG. 4 is a flowchart illustrating the process for introduction of an information tag;
FIG. 5 is a diagram illustrating the procedure for introducing an information tag;
FIG. 6 is a diagram illustrating negative selection;
FIG. 7 is a diagram illustrating positive selection;
FIG. 8 is a diagram illustrating how to remove the drug tolerant gene for positive selection;
FIG. 9 is a flowchart illustrating the process for acquisition of an information tag;
FIG. 10 is a diagram illustrating how to acquire the base sequence encoded with an information tag from an organism into which the information tag has been introduced;
FIG. 11 is a diagram showing an example of the recognition site of the restriction endonuclease;
FIG. 12 is a diagram showing the structure of a DNA;
FIG. 13 is a diagram showing the position of the DNA shown in FIG. 12 into which an information tag is introduced;
FIG. 14 is a diagram showing the unit of introduction of the base sequence to be introduced;
FIG. 15 is a diagram showing a partial structure of the base sequence to be introduced;
FIG. 16 is a diagram showing another example of the structure of a DNA;
FIG. 17 is a diagram showing the position of the DNA shown in FIG. 16 into which an information tag is introduced;
FIG. 18 is a diagram showing the unit of introduction of the base sequence to be introduced;
FIG. 19 is a diagram showing a partial structure of the base sequence to be introduced;
FIG. 20 is a flowchart illustrating in detail the process of Step S102 in FIG. 4;
FIG. 21 is a diagram showing an example of the information data to be introduced;
FIG. 22 is a diagram showing correspondence between the species of base and the binary data;
FIG. 23 is a diagram showing the construction of 8-bit data;
FIG. 24 is a flowchart illustrating in detail the process of Step S304 in FIG. 20;
FIG. 25 is a flowchart which continues from FIG. 24, illustrating in detail the process of Step S304 in FIG. 20;
FIG. 26 is a diagram showing the position into which the error correction code is inserted;
FIG. 27 is a diagram showing the method for selecting the error correction code;
FIG. 28 is a diagram showing the position into which the buffer sequence is inserted;
FIG. 29 is a flowchart illustrating in detail the process of Step S305 in FIG. 20;
FIG. 30 is a diagram showing an example of the buffer sequence;
FIG. 31 is a diagram showing the flow of determining the buffer sequence;
FIG. 32 is a flowchart illustrating the process of preparing homologous sequence database;
FIG. 33 is a flowchart illustrating in detail the process of Step S603 in FIG. 32;
FIG. 34 is a diagram illustrating how to perform homology search;
FIG. 35 is a diagram showing an example of the homologous sequence pattern list;
FIG. 36 is a flowchart illustrating in detail the process of Step S309 in FIG. 20;
FIG. 37 is a diagram illustrating how to search for the position of introduction;
FIG. 38 is a flowchart illustrating in detail the process of Step S205 in FIG. 9;
FIG. 39 is a diagram illustrating error correction;
FIG. 40 is another diagram illustrating error correction;
FIG. 41 is a further another diagram illustrating error correction;
FIG. 42 is a diagram showing another example of the position into which an information tag is introduced;
FIG. 43 is a flowchart illustrating the process of detecting the creator's information tag;
FIG. 44 is a diagram illustrating how to detect the information tag; and
FIG. 45 is a block diagram showing an example of the construction of a personal computer to which the present invention is applied.

### DETAILED DESCRIPTION OF THE PREFERRED EMBODIMENTS

A description is made below of the best mode for carrying out the present invention. There is a following correspondence between the disclosure of the invention and the embodiment of the invention. There may exist some embodiments which are mentioned in the specification but are not mentioned in this section as those corresponding to the disclosure of the invention. It does not mean that such embodiments do not correspond to the disclosure of the invention. Conversely, even though some embodiments may be mentioned as corresponding to the disclosure of the invention, it does not necessarily mean that such embodiments do not correspond to the disclosure of any other invention than the present invention.

Moreover, the description herein does not embrace the entire invention disclosed in this specification. In other words, the description herein is limited to the disclosure of the invention mentioned in this specification, and hence it does not intend to deny the presence of any other invention which will appear in the future as the result of division of application or amendment of application.

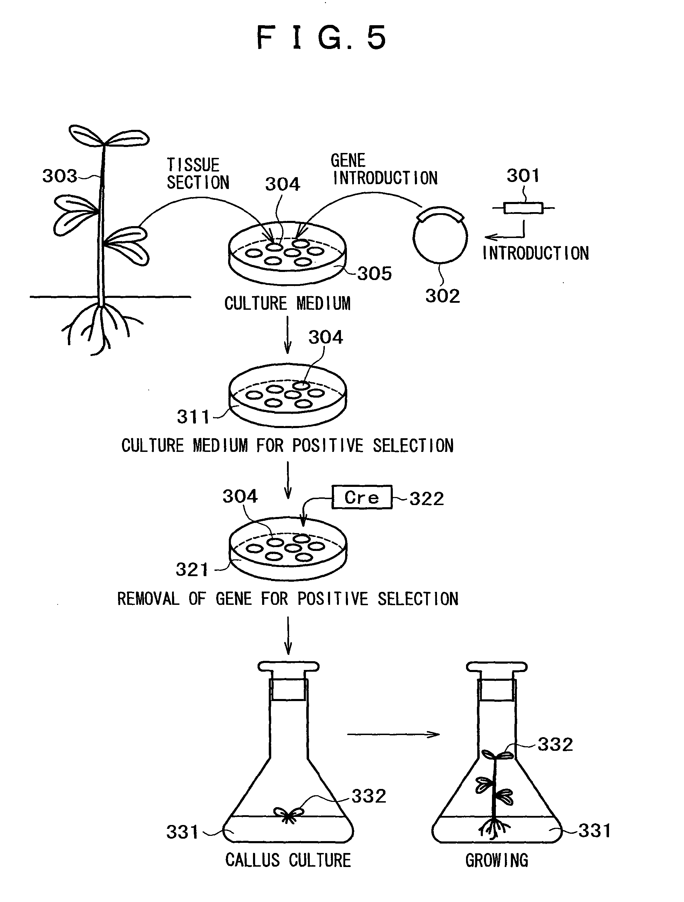

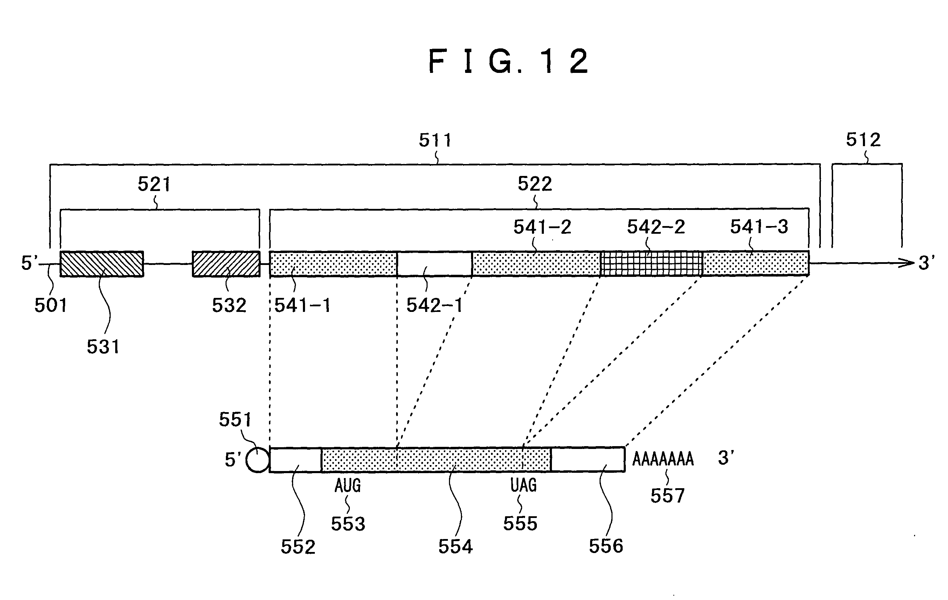

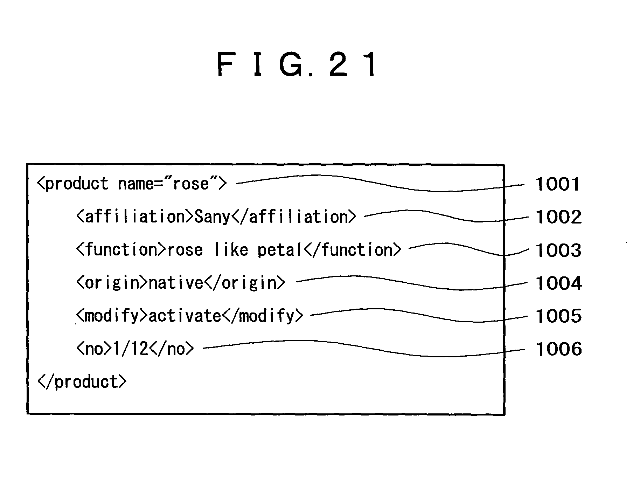

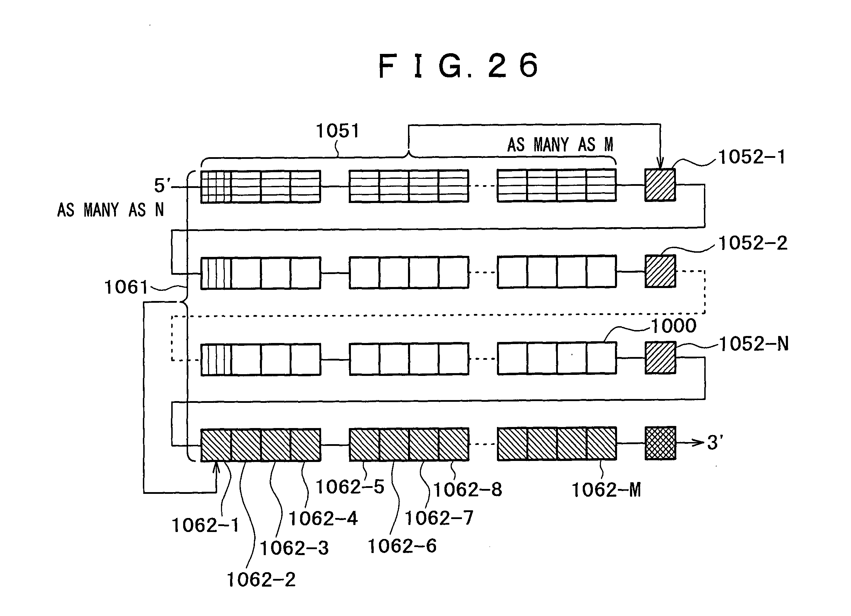

The present invention provides a DNA. This DNA is encoded with information to be introduced (such as information data shown in FIG. 21) into a biogenic DNA (such as DNA 501 shown in FIG. 12) of a genetically manipulated organism (such as plant 303 shown in FIG. 5) and which has an error-correcting base inserted thereinto (such as correcting codes 1052-1 to 1052-N and correcting codes 1062-1 to 1062-M shown in FIG. 26).

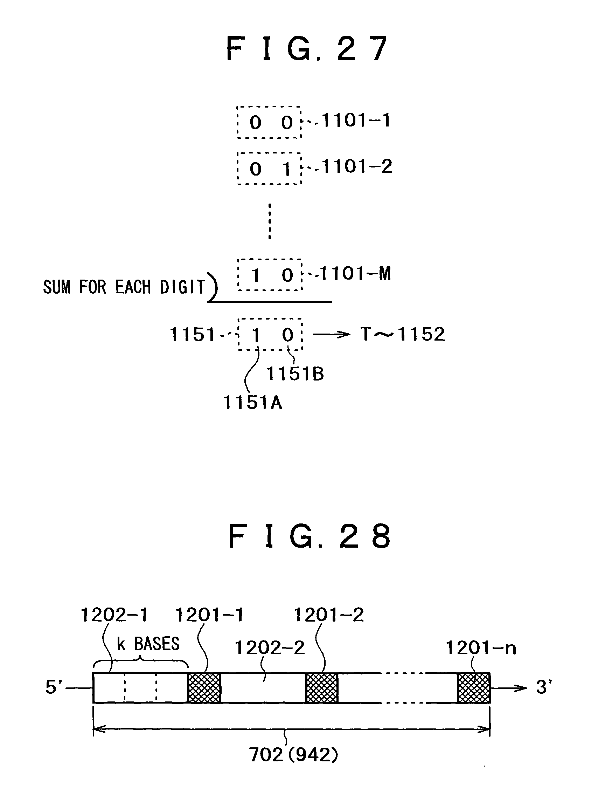

The present invention provides a DNA. This DNA may have an additional base for buffering (such as buffer sequence 1201-1 to 1201-n shown in FIG. 28) so that it decreases in homology to the base sequence of DNA of more than one species of organism.

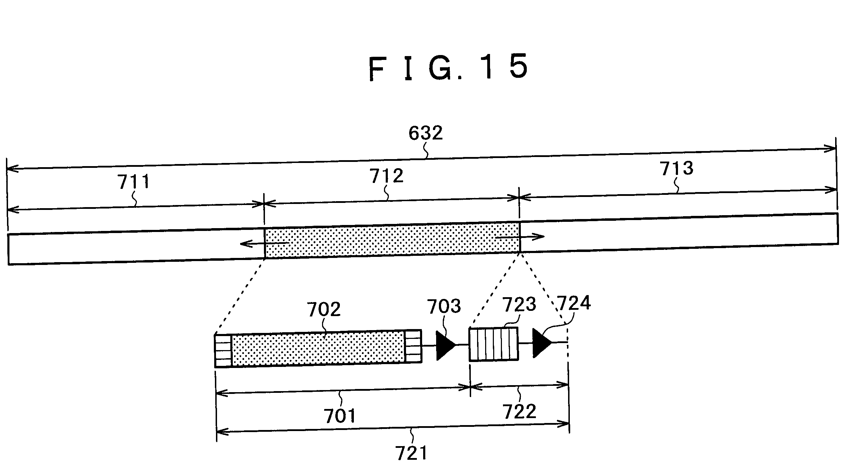

The present invention provides a DNA. This DNA may additionally have a drug tolerant gene for positive selection (such as gene 723 shown in FIG. 15).

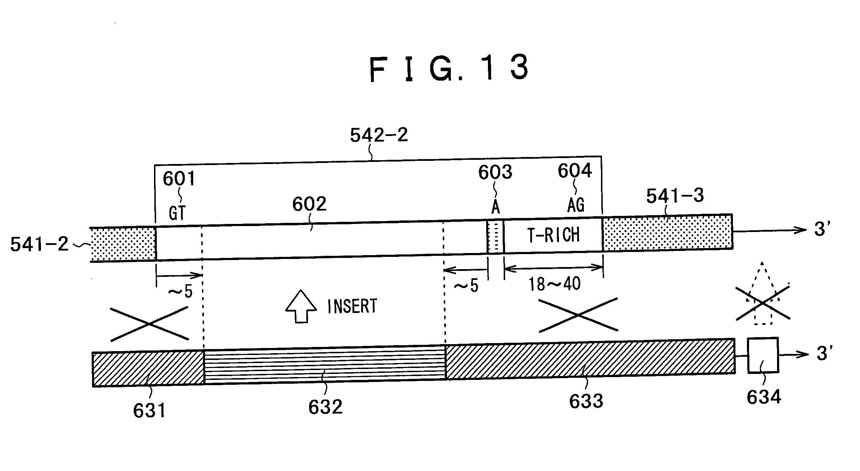

The present invention provides a DNA. This DNA may additionally have a drug tolerant gene for negative selection (such as gene 634 shown in FIG. 13).

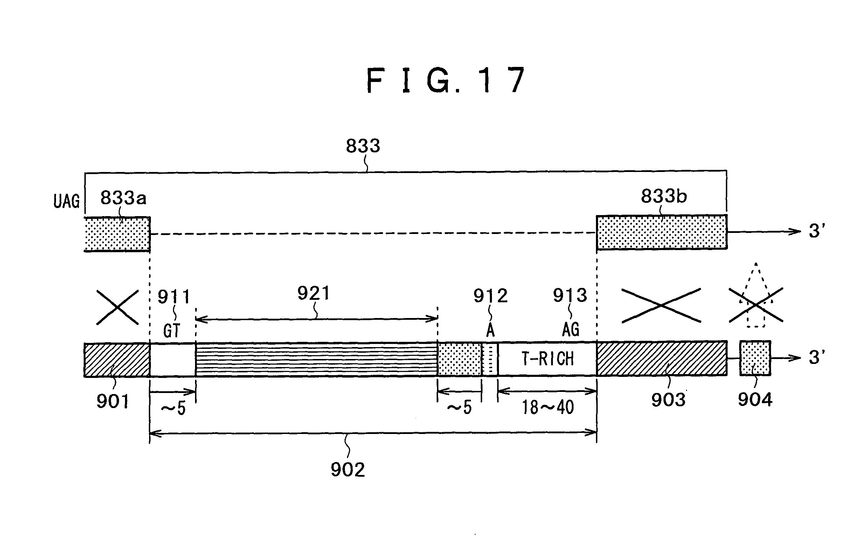

The present invention provides a DNA. This DNA may additionally have the base of intron sequence which is recognized as the intron region by the genetically manipulated organism. (The base of intron sequence is indicated by the region covering five bases counted from GT 911 and by the region extending from the fifth base counted upstream from A 912 to AG 913, as shown in FIG. 17.)

The present invention provides a vector for gene introduction. This vector (such as vector 302 shown in FIG. 5) contains a DNA which is encoded with information to be introduced (such as information data shown in FIG. 21) into a biogenic DNA (such as DNA 501 shown in FIG. 12) of a genetically manipulated organism (such as plant 303 shown in FIG. 5) and which has an error-correcting base inserted thereinto (such as correcting codes 1052-1 to 1052-N and correcting codes 1062-1 to 1062-M shown in FIG. 26).

The present invention provides a cell for gene introduction. This cell contains a DNA which is encoded with information to be introduced (such as information data shown in FIG. 21) into a biogenic DNA (such as DNA 501 shown in FIG. 12) of a genetically manipulated organism (such as plant 303 shown in FIG. 5) and which has an error-correcting base inserted thereinto (such as correcting codes 1052-1 to 1052-N and correcting codes 1062-1 to 1062-M shown in FIG. 26).

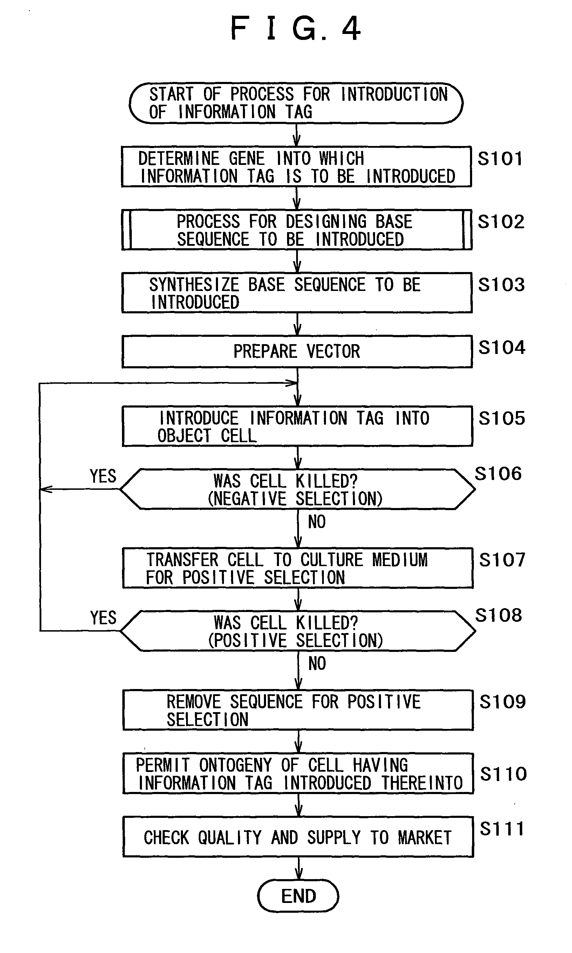

The present invention provides a method for introduction of information. This method includes a step of designing the base sequence (such as Step S102 shown in FIG. 4) of a DNA to be introduced into a biogenic DNA (such as DNA 501 shown in FIG. 12), the DNA being encoded with information to be introduced (such as information data shown in FIG. 21) into a biogenic DNA of a genetically manipulated organism (such as plant 303 shown in FIG. 5) and having an error-correcting base inserted thereinto (such as correcting codes 1052-1 to 1052-N and correcting codes 1062-1 to 1062-M shown in FIG. 26), a step of synthesizing (such as Step S103 shown in FIG. 4) a DNA having the thus designed base sequence, and a step of introducing (such as Step S105 shown in FIG. 4) the thus synthesized DNA into a genetically manipulated gene.

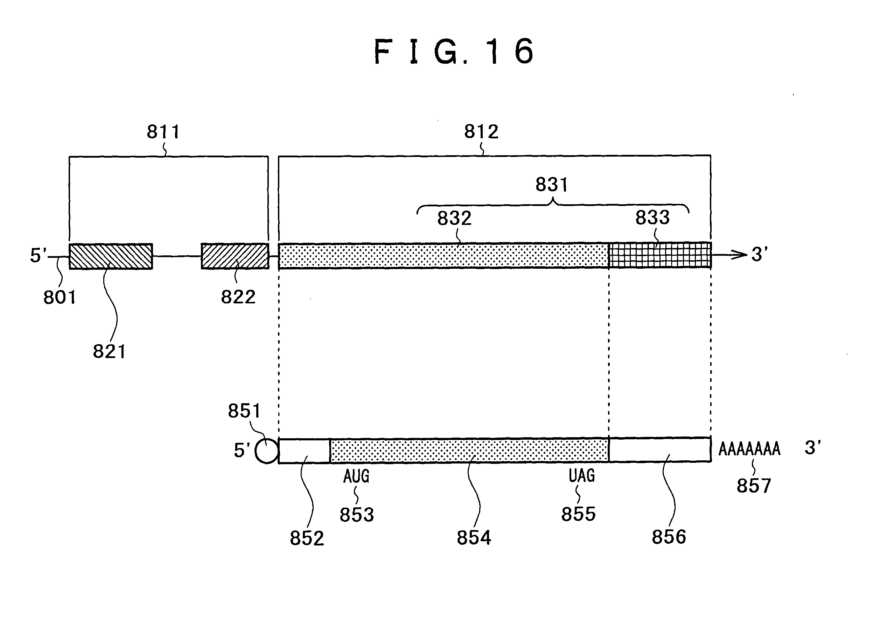

The present invention provides a method for introduction of information. This method may be accomplished in such a way that the DNA is introduced into a region not encoded with protein, out of the base sequence of the genetically manipulated gene (such as intron region 542-2 shown in FIG. 12 or base sequence 833 shown in FIG. 16).

The present invention provides a method for introduction of information. This method may be accomplished in such a way that the region not encoded with protein is an intron region (such as intron region 542-2 shown in FIG. 12).

The present invention provides a method for introduction of information. This method may be accomplished in such a way that the region not encoded with protein is a region of the base sequence complementary to the untranslated region (such as base sequence 833 shown in FIG. 16) which is not translated into the protein, of the base sequence of mRNA to which the gene has been transcribed, out of the biogenic DNA.

The present invention provides a method for introduction of information. This method may be accomplished in such a way that the base sequence of the DNA to be introduced into the biogenic DNA further contains an intron sequence which is recognized as the intron region by the genetically manipulated organism. (The base of intron sequence is indicated by the region covering five bases counted from GT 911 and by the region extending from the fifth base counted upstream from A 912 to AG 913, as shown in FIG. 17.)

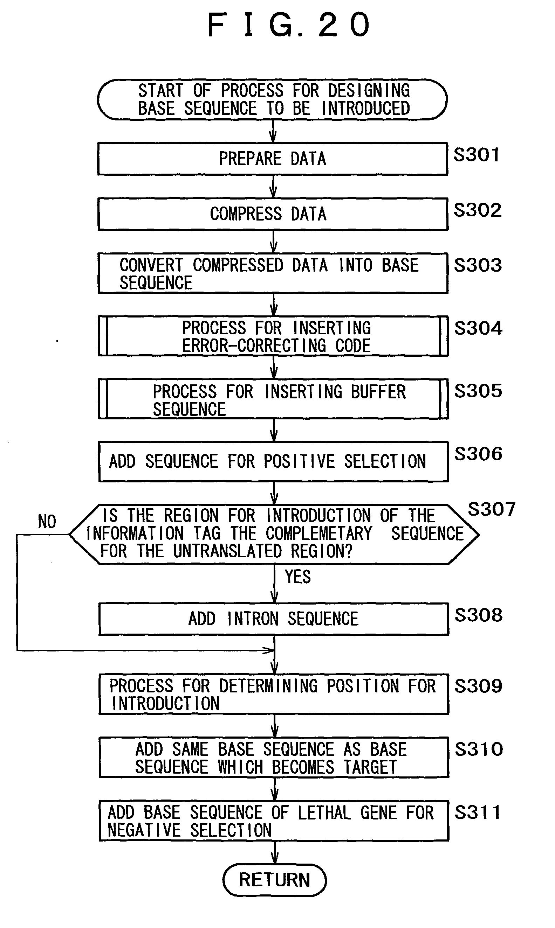

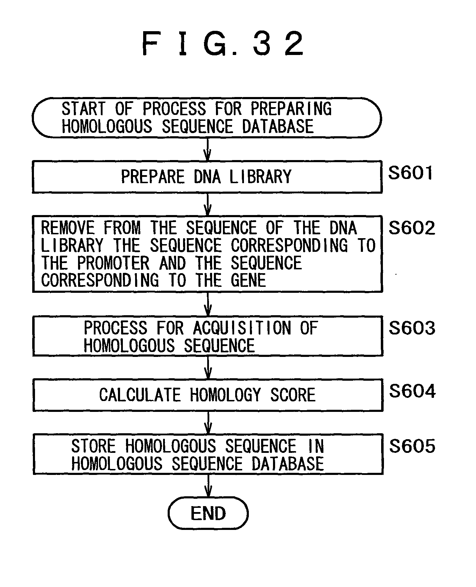

The present invention provides a method for introduction of information. This method may include a step of acquiring a base sequence pattern similar to that present in more than one species of organisms according to the base sequence of DNA of more than one species of organisms before introducing the synthesized DNA into the genetically manipulated gene (such as process for preparing database of homologous sequence shown in FIG. 32), a step of selecting a fragment of base sequence having the lowest similarity to the base sequence pattern from the region in which the genetically manipulated gene is not encoded with protein (such as Step S309 shown in FIG. 20), and a step of introducing the synthesized DNA into the fragment selected as the fragment having the lowest similarity to the base sequence pattern at the time of introducing the DNA into the genetically manipulated gene (such as Step S105 shown in FIG. 4).

The present invention provides a method for introduction of information. This method may include a step of acquiring a base sequence pattern similar to that present in more than one species of organisms according to the base sequence of DNA of more than one species of organisms before designing the base sequence of the DNA to be introduced into the biogenic DNA (such as process for preparing database of homologous sequence shown in FIG. 32), and a step of inserting a buffer base sequence so that the similarity to the acquired base sequence pattern becomes least, thereby designing the the base sequence of the DNA to be introduced into the biogenic DNA at the time of designing the base sequence of the DNA to be introduced into biogenic DNA (such as Step S305 shown in FIG. 20).

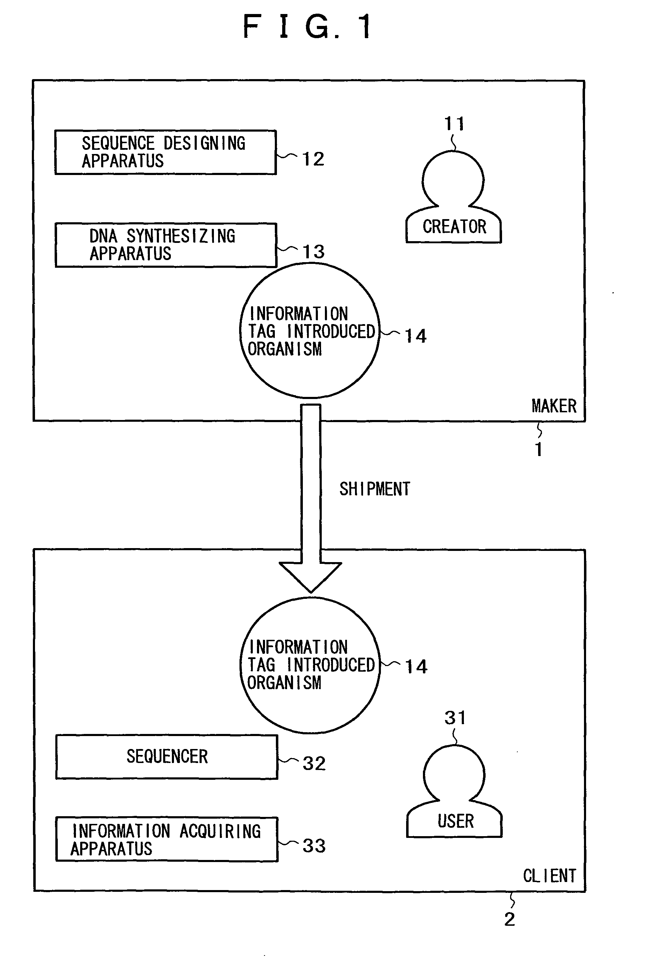

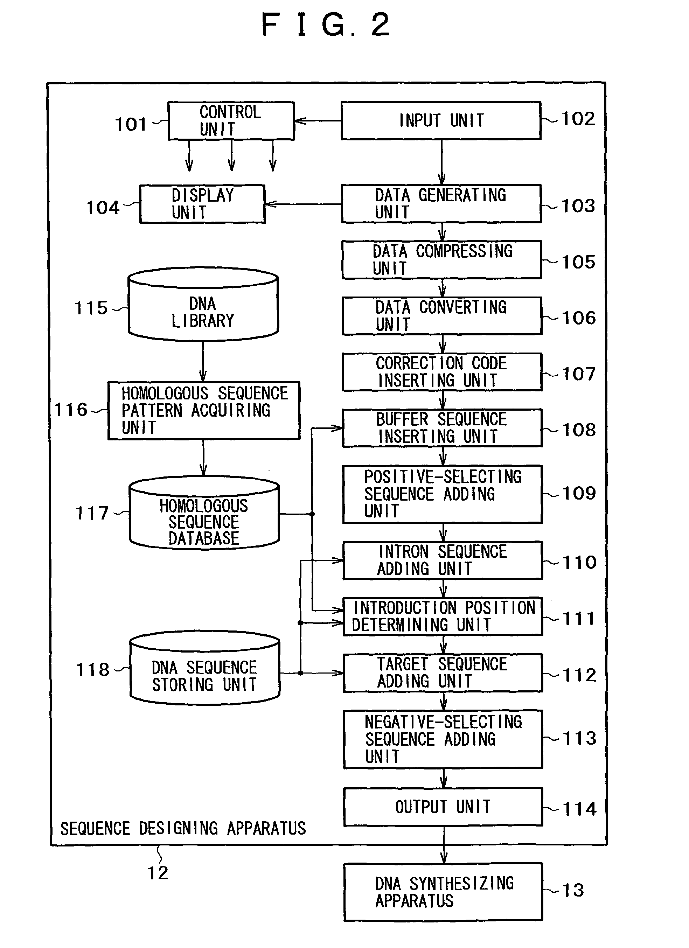

The present invention provides a data processing apparatus. This data processing apparatus (such as sequence designing apparatus 12 shown in FIG. 1) includes means (such as data converting part 106 shown in FIG. 2) to convert binary data containing information (such as information data shown in FIG. 21) to be introduced into a biogenic DNA (such as DNA 501 shown in FIG. 12) of a genetically manipulated organism (such as plant 303 shown in FIG. 5) into base sequence data and means (such as correction code inserting unit 107 shown in FIG. 2) to insert base data for error correction (such as correcting codes 1052-1 to 1052-N and correcting codes 1062-1 to 1062-M shown in FIG. 26) into the base sequence data obtained by above-mentioned converting means.

The present invention provides a data processing method. This data processing method includes a step (such as Step S303 shown in FIG. 20) of converting binary data containing information (such as information data shown in FIG. 21) to be introduced into a biogenic DNA (such as DNA 501 shown in FIG. 12) of a genetically manipulated organism (such as plant 303 shown in FIG. 5) into base sequence data and a step (such as Step S304 shown in FIG. 20) of inserting base data for error correction (such as correcting codes 1052-1 to 1052-N and correcting codes 1062-1 to 1062-M shown in FIG. 26) into the base sequence data obtained by aforementioned converting step.

The present invention provides a program similar to that used for information processing.

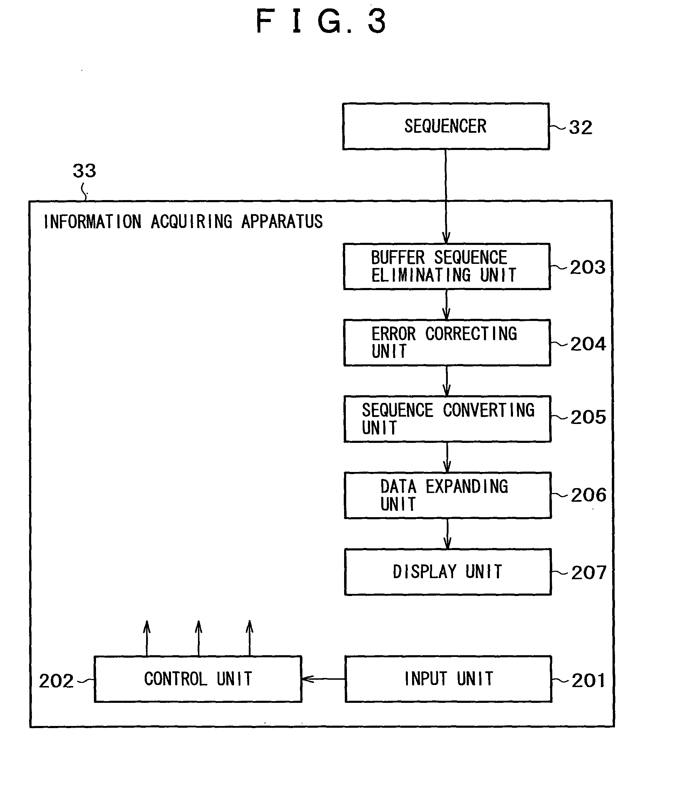

The present invention provides a data processing apparatus. This data processing apparatus (such as information acquiring apparatus 33 shown in FIG. 1) includes means (such as error correcting unit 204 shown in FIG. 3) to correct errors in base sequence data according to the base sequence for error correction (such as correcting codes 1052-1 to 1052-N and correcting codes 1062-1 to 1062-M shown in FIG. 26) inserted into the base sequence data, and means (such as sequence converting unit 205 shown in FIG. 3) to convert the base sequence data, which has been error-corrected by the correcting means (such as error correcting unit 204 shown in FIG. 3), into binary data.

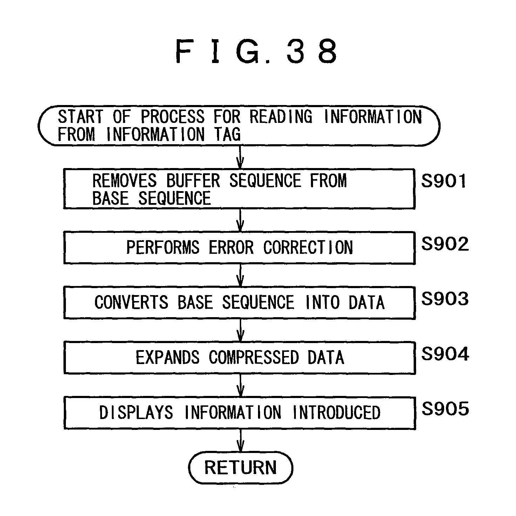

The present invention provides a data processing method. This data processing method includes a step (such as Step 902 shown in FIG. 38) of correcting errors in base sequence data according to the base sequence for error correction (such as correcting codes 1052-1 to 1052-N and correcting codes 1062-1 to 1062-M shown in FIG. 26) inserted into the base sequence data, and a step (such as Step S902 shown in FIG. 38) to convert the base sequence data, which has been error-corrected by the correcting step (such as Step S903 shown in FIG. 38), into binary data.

The present invention provides a program similar to that used for information processing.

The embodiment of the present invention will be described below with reference to the accompanying drawings.

FIG. 1 shows an example of the construction of the information processing system to which the present invention is applied.

In FIG. 1, the creator 11 of the maker 1 prepares a information tag introduced organism 14 having an information tag introduced thereinto. To achieve this object, the creator 11 introduces a DNA encoded with desired information (referred to as information tag hereinafter) into a DNA of a living organism on which genetic manipulation is to be performed (referred to as biogenic DNA), by using the sequence designing apparatus 12, DNA synthesizing apparatus 13, and other apparatus and chemicals (not shown). Incidentally, the creator 11 introduces the maker name, the product name of the information tag introduced organism 14, and the DNA encoded with information (information tag) such as the content of genetic manipulation performed on the living organism, into a DNA of a living organism on which genetic manipulation is to be performed. (The information to be introduced into a living organism is referred to as information to be introduced hereinafter.)

The sequence designing apparatus 12, upon receipt of the information to be introduced from the creator 11, designs the base sequence encoded with the entered information and outputs the designed base sequence (referred to as sequence design data hereinafter) to the DNA synthesizing apparatus 13.

The DNA synthesizing apparatus 13 synthesizes a DNA with the base sequence corresponding to the sequence design data entered from the sequence designing apparatus 12, and it offers the synthesized DNA (or the information tag) to the creator 11. The creator 11 introduces the information tag, which has been synthesized by the DNA synthesizing apparatus 13, into the cell, into which the gene is to be introduced, by means of a gene introducing vector such as virus vector.

Once the creator 11 has prepared the information tag introduced organism 14, it is subsequently shipped to the client 2, such as research institutes and enterprises.

Upon receipt, from the maker 1, of the information tag introduced organism 14, the user 31 of the client 2 utilizes it so as to develop and produce foods and medicines. The user 31 may also acquire information from the information tag introduced organism 14 by using the sequencer 32 and the information acquiring apparatus 33 and other apparatus and medicines (not shown).

The sequencer 32 identifies the base sequence of the nucleic acid (containing the information tag) which has been extracted from the DNA of the information tag introduced organism 14.

The information acquiring apparatus 33 reads information from the base sequence entered from the sequencer 32 and submits it to the user 31.

FIG. 2 shows an example of the internal constitution of the sequence designing apparatus 12 shown in FIG. 1.

In FIG. 2, the control unit 101 controls the action of each unit in the sequence designing apparatus 12 according to the operating information supplied from the previously stored program or the input unit 102. The input unit 102 includes a keyboard, pointing device, etc. It receives input for operation from the creator 11 and supplies the operating information (corresponding to the operation entered) to the control unit 101 and the data generating unit 103. Incidentally, the operation entered from the creator 11 includes the operation to specify the action of the sequence designing apparatus 12 and the operation to enter the information to be introduced.

The data generating unit 103 generates and modifies data containing the information to be introduced according to the control of the control unit 101 and the operating information supplied from the input unit 102, and then supplies it to the display unit 104. Also, as soon as the data generating unit 103 receives from the control unit 101 an instruction to fix the information to be introduced into the organism, it supplies the data compressing unit 105 with the data containing the information to be introduced.

The display unit 104 may be an LCD (Liquid Crystal Display) or a CRT (Cathode Ray Tube). It displays the information to be introduced into the living organism according to the data containing the information to be introduced, which has been supplied from the data generating unit 103. The creator 11 enters or modifies the information to be introduced while watching it on the display unit 104.

The data compressing unit 105 receives from the data generating unit 103 the data of information to be introduced and then compresses the received data according to the previously established encoding system. The compressed data is supplied to the data converting unit 106. This procedure reduces the amount of data to be introduced into the living organism and hence minimizes the effect on the living organism. The compressed data which is output from the data compressing unit 105 is binary data.

Upon receipt of the compressed data (in the form of binary data) from the data compressing unit 105, the data converting unit 106 converts it into the sequence of four bases T (thymine), A (adenine), C (cytosine), and G (guanine), according to the previously established correspondence. In other words, the data converting unit 106 divides the binary data into sections each consisting of two digits and allocates each section to its corresponding base. In this way conversion from the compressed data into the base sequence data is accomplished. For example, the data converting unit 106 converts two digits of binary data into the base sequence data as follows. "00" into A (adenine), "01" into G (guanine), "11" into C (cytosine), and "10" into T (thymine). In this way it is possible to convert all of the four patterns of two-digit binary number ("00", "01", "11", and "10") into their corresponding bases. In the above-mentioned case, the binary data "0011100110" is converted into the base sequence data of "ACTGT".

The data converting unit 106 divides the compressed binary data into two-digit sections and converts them into their corresponding bases. In this way it generates the base sequence data and supplies it to the correction code inserting unit 107. Incidentally, the correspondence between the two-digit binary number and the base sequence is a mere example, and any other correspondence may be employed.

The correction code inserting unit 107 inserts an error correction code into the base sequence data supplied from the data converting unit 106 and then supplies the base sequence data (having the error correction code inserted thereinto) to the buffer sequence inserting unit 108. The effect of this procedure is that even though the bases of the base sequence of the information tag which has been introduced into a living organism is partly converted by other bases, it is possible to make correction and take out correctly the information which has been introduced.

The buffer sequence inserting unit 108 receives from the correction code inserting unit 107 the base sequence data (having the error correction code inserted thereinto), and then inserts it into the buffer sequence and supplies the base sequence data (having the buffer sequence inserted thereinto) to the positive-selecting sequence adding unit 109. Incidentally, the buffer sequence inserting unit 108 references the homologous sequence data stored in the homologous sequence database 117, thereby determining the buffer sequence to be inserted into the base sequence data which has been supplied from the correction code inserting unit 107. The effect of inserting the buffer sequence into the base sequence data is that it is possible to prevent the information tag from becoming similar to the sequence which is highly likely to be important for the living organism. As the result, it is possible to prevent the information tag from adversely affecting the living organism.

The positive-selecting sequence adding unit 109 receives from the buffer sequence inserting unit 108 the base sequence data having the buffer sequence inserted thereinto, and then adds to it the base sequence of a drug tolerant gene for positive selection which is used to test to see whether or not the information tag has been inserted into the living organism.

The intron sequence adding unit 110 receives from the positive-selecting sequence adding unit 109 the base sequence data having added thereto the base sequence of a drug tolerant gene for positive selection. In this case, the intron sequence adding unit 110 references the base sequence of the gene for introduction of the information tag (the base sequence being stored in the DNA sequence storing unit 118), thereby to determine whether or not the intron sequence should be added to the base sequence data supplied form the positive-selecting sequence adding unit 109. If the intron sequence adding unit 110 determines that the intron sequence should be added to the base sequence data, then the intron sequence adding unit 110 adds the intron sequence to the base sequence which is specified by the base sequence data having the sequence for positive selection added thereto, the base sequence data being supplied from the positive-selecting sequence adding unit 109, and then supplied the added base sequence data to the introduction position determining unit 111. On the other hand, if the intron sequence adding unit 110 determines that the intron sequence should not be added to the base sequence data, then the intron sequence adding unit 110 supplies the base sequence data having the sequence for positive selection added thereto (which has been supplied from the positive-selecting sequence adding unit 109) as such to the introduction position determining unit 111. In this case, the intron sequence is not added to the base sequence specified by the base sequence data.

When the information tag is inserted into the biogenic DNA, there are two cases: one is that the information tag is inserted into the intron of the biogenic DNA, and the other is that the information tag is inserted into the complementary sequence for the untranslated region of mRNA in the biogenic DNA. In the latter case, the sequence of the information tag is also transcribed into mRNA when the gene into which the information tag has been inserted is transcribed and mRNA is generated. The result of adding the intron sequence to the information tag is that even though the sequence of the information tag has been transcribed into mRNA, the sequence corresponding to the information tag is removed by splicing and hence this eliminates the effect on the living organism.

The introduction position determining unit 111 specifies the length (the number of units) of the base sequence according to the base sequence data supplied from the intron sequence adding unit 110. And, it references the homologous sequence data (which is stored in the homologous sequence database 117) and the base sequence of the biogenic DNA of the organism as the object of genetic manipulation (which is stored in the DNA sequence storing unit 118), thereby to determine the position into which the information tag should be inserted. This position is one at which the insertion of the information tag into intron (or the complementary sequence for the untranslated region) will affect least the living organism. The introduction position determining unit 111 receives the base sequence data from the intron sequence adding unit 110 and supplies it to the target sequence adding unit 112. It also supplies the target sequence adding unit 112 with the information which indicates the position at which the determined information tag should be introduced.

The target sequence adding unit 112 receives the base sequence data from the introduction position determining unit 111 and adds the sequence for which the homology recombination is made to take place to the received base sequence data. In other words, for introduction of the information tag into the biogenic DNA by homology recombination, it is necessary that the base of the same base sequence as that upstream and downstream from the position on the biogenic DNA at which the information tag is introduced should be added to the upstream and downstream sides of the information tag. Thus, the target sequence adding unit 112 uses the introduction position information supplied from the introduction position determining unit 111, thereby to read the base sequence corresponding to the upstream and downstream sides of the position at which the information tag is introduced out of the base sequence of the living organism (which is stored in the DNA sequence storing unit 118) on which gene manipulation is performed, and then the target sequence adding unit 112 adds the base sequence to the upstream and downstream sides of the base sequence data supplied from the introduction position determining unit 111. The target sequence adding unit 112 supplies the negative-selecting sequence adding unit 113 with the base sequence data to which is added the base sequence of the biogenic DNA corresponding to the upstream and downstream sides of the position at which the information tag is introduced.

After receiving the base sequence data from the target sequence adding unit 112, the negative-selecting sequence adding unit 113 adds to the base sequence data the base sequence of the negative-selecting lethal gene to test whether or not the information tag has been introduced into the living organism, and subsequently the negative-selecting sequence adding unit 113 supplies the result to the output unit 114.

After receiving the base sequence data supplied from the negative-selecting sequence adding unit 113, the output unit 114 outputs it (as the sequence design data) to the DNA synthesizing apparatus 13. The DNA synthesizing apparatus 13 synthesizes an actual DNA based on the sequence design data entered from the output unit 114, and then it submits the resulting DNA to the creator 11.

The DNA library 115 stores the base sequence of the genome of more than one species of organisms. It is read out by the homologous sequence pattern acquiring unit 116 as the occasion may demand. Incidentally, it is desirable that the DNA library 115 should be made to store the base sequence of the genome of the species of the organism akin to the organism on which gene manipulation is performed.

The homologous sequence pattern acquiring unit 116 prepares a list showing how one base sequence pattern resembles another one in the base sequence of the genome of more than one species of organisms which is stored in the DNA library 115, and then the homologous sequence pattern acquiring unit 116 causes the homologous sequence database 117 to store the resulting list. In other words, although organisms evolved as the result of alteration in the base sequence of DNA, the base sequence important for their survival (such as the base sequence encoding proteins essential for survival) remained comparatively unchanged regardless of species of organisms (or remained similar among different species). This suggests that the base sequences similar among a plurality of species are more important for their survival. So, the homologous sequence pattern acquiring unit 116 specifies as the homologous sequence pattern the base sequence pattern appearing in common in the genome of more than one species of organism. Then, the homologous sequence pattern acquiring unit 116 obtains an average score showing how the homologous sequence pattern resembles among different species of organisms and also obtains how frequently the homologous sequence pattern appears. The average score multiplied by the frequency gives the homology score, which is an index indicating the importance of the homologous sequence pattern. The homologous sequence pattern acquiring unit 116 stores the calculated homology score (associated with the homologous sequence pattern) in the homologous sequence database 117.

The homologous sequence database 117 stores the homology score (associated with the homologous sequence pattern) and supplies it to the buffer sequence inserting unit 108 and the introduction position determining unit 111 as the occasion demands.

The DNA sequence storing unit 118 stores the base sequence data indicating the base sequence of the genome of the species of the organism on which gene manipulation is performed. The base sequence data of the genome of the species of the organism on which gene manipulation is performed, which is stored in the DNA sequence storing unit 118, is read out by the intron sequence adding unit 110, the introduction position determining unit 111, and the target sequence adding unit 112, as the occasion demands.

Next, FIG. 3 shows an example of the construction of the information acquiring apparatus 33 shown in FIG. 1.

In FIG. 3, the input unit 201 includes a keyboard and a pointing device. The input unit 201 receives input for operation from the user 31, and then it supplies the control unit 202 with information corresponding to input for operation. The control unit 202 controls actions of each unit in the information acquiring apparatus 33 according to the previously stored program and the information for operation supplied from the input unit 201.

The buffer sequence eliminating unit 203 receives the base sequence data of the information tag from the sequencer 32, eliminates the buffer sequence from the supplied base sequence data, and supplies the error-correcting unit 204 with the base sequence data from which the buffer sequence has been eliminated.

The error correcting unit 204 receives the base sequence data from the buffer sequence eliminating unit 203 and corrects errors in the base sequence data by using the error correction code contained in the base sequence data. It further supplies the error-corrected base sequence data to the sequence converting unit 205.

Upon receipt of the base sequence data from the error-correcting unit 204, the sequence converting unit 205 converts it into binary data. In other words, since the base sequence data is the data which has been converted from binary data (compressed data) by the data converting unit 106 shown in FIG. 2, the sequence converting unit 205 converts the base sequence data into binary data in correspondence with conversion from the binary data (produced by the data converting unit 106) into the base sequence data. For example, if the data converting unit 106 converts binary two-digit data into base sequence data such that "00" into A (adenine), "01" into G (guanine), "11" into C (cytosine), and "10" into T (thymine), as mentioned above, then the sequence converting unit 205 converts the base sequence data into binary two-digit data such that A (adenine) into "00", G (guanine) into "01", C (cytosine) into "11", and T (thymine) into "10". As the result of this process, the base sequence data is converted into binary data (or compressed data). After generating the compressed data by conversion from the base sequence data, the sequence converting unit 105 supplies it to the data expanding unit 206.

After receiving the compressed data from the sequence converting unit 205, the data expanding unit 206 expands it according to the decoding system corresponding to the coding system of the data compressing unit 105 shown in FIG. 2. The data expanding unit 206 further outputs the information for introduction to the display unit 207 according to the expanded data. In other words, the expanded data corresponds to the data prepared by the data generating unit 103 shown in FIG. 2 and it contains the information to be introduced. Therefore, the data expanding unit 206 causes the display unit 207 to display the information for introduction (such as the maker name, the product name of the information tag introduced organism 14, and the content of gene manipulation).

The display unit 207 may be an LCD or CRT. It displays the information for introduction supplied from the data expanding unit 206. The information for introduction which is displayed on the display unit 207 permits the user 31 to know information, such as the name of the maker who has prepared the information tag introduced organism 14, the product name, and the content of gene manipulation performed on the information tag introduced organism 14.

The following is concerned with the process for introducing an information tag into an organism which has undergone gene manipulation. The flowchart shown in FIG. 4 should be referenced together with FIGS. 5 to 8.

In Step S101 shown in FIG. 4, the creator 11 determines the manipulated gene as the gene into which the information tag is introduced and specifies the gene locus of the gene into which the information tag is introduced. (The gene locus is the position on the gene map.)

In Step S102, the sequence designing apparatus 12 designs, according to operation by the creator 11, the base sequence containing the base sequence of the information tag to be introduced. The sequence designing apparatus 12 supplies the thus designed base sequence data to the DNA synthesizing apparatus 13.

In Step S103, the DNA synthesizing apparatus 13, which has received the sequence design data from the sequence designing apparatus 12, synthesizes the DNA (containing the information tag) which has the base sequence specified by the sequence design data. At the upper right part of FIG. 5 is shown the synthesized DNA which is the base sequence 301 to be introduced.

In Step S104, the creator 11 prepares a vector for gene introduction containing the DNA (the base sequence 301 to be introduced) which has been synthesized in Step S103. This vector is used to introduce into the living organism the DNA which has been synthesized in Step S103. At the upper right part of FIG. 5 is shown the vector 302 containing the synthesized DNA. This is the vector for gene introduction. The creator 11 prepares this vector 302.

In Step 105, the creator 11 introduces the information tag into the object cell by using the vector 302. In the upper left part of FIG. 5 is shown the plant 303 into which the information tag is to be introduced. It is assumed that the plant 303 has already undergone gene manipulation. As shown in the central upper part of FIG. 5, the tissue section 304 of the plant 303 is infected with the vector 302 so that the information tag is introduced into the cell originating from the plant 303. Incidentally, the tissue section 304, which has been brought into contact with the vector 302, is subsequently cultured on the culture medium 305.

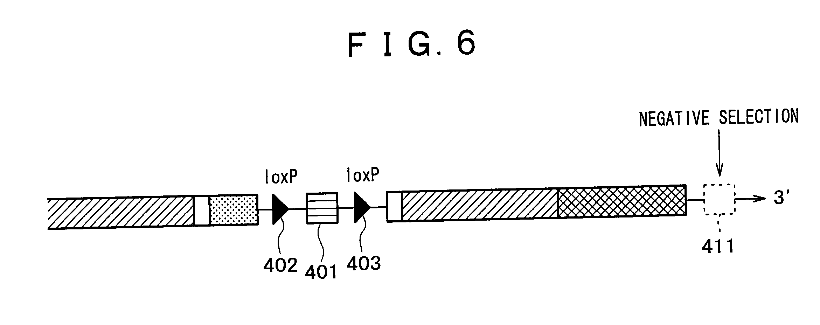

The DNA to be introduced into the object cell contains the lethal gene for negative selection 411 as shown in FIG. 6. In other words, the numeral 411 in FIG. 6 denotes the lethal gene for negative selection. If the base sequence of the information tag is not taken up by the biogenic DNA of the object for introduction, and if only the portion of the information tag is accurately taken up by homology recombination, then the cell survives; however, if the lethal gene for negative selection 411 (which is other parts than the information tag) is also taken up, then the cell does not survive because the lethal gene for negative selection 411 expresses in the cell. (This is referred to as negative selection.) Also in FIG. 6, the numeral 401 denotes the drug tolerant gene for positive selection, and the numerals 402 and 403 denote the loxP sequence. If the drug tolerant gene for positive selection 401 and the loxP sequences 402 and 403 are taken up into the biogenic DNA of the object cell, then the cell can survive even in a culture medium dosed with a specific drug. (This is referred to as positive selection.)

Thus, in Step S106, the creator 11 determines whether or not the tissue section 304 on the culture medium 305 was killed. If the tissue section 304 was killed (because the lethal gene for negative selection 411 was taken up into the cell) or if the introduction of the information tag into the cell failed, then the process returns to Step S105 so that steps after Step S105 are repeated.

If the cell was not killed in Step S106 (or if the base sequence of the information was not taken up into the biogenic DNA of the object for introduction or if only the part of the information tag was accurately taken up due to homology recombination), then the process proceeds to Step S107.

In Step S107, the creator 11 transfers the tissue section 304 from the culture medium 305 to the culture medium for positive selection 311 shown in FIG. 5 and continues culture. The culture medium 311 for positive selection 311 contains a specific drug so that only the cell in which the drug tolerant gene for positive selection expresses can survive. As mentioned above, if the drug tolerant gene 401 is taken up into the biogenic DNA of the object cell, then the cell can survive even in the culture medium for positive selection 311 which is dosed with a specific drug. On the other hand, it the drug tolerant gene 401 is not taken up into the biogenic DNA of the object cell, then the cell cannot survive in the culture medium 311 dosed with a specific drug. Thus, in Step S108, the creator 11 determines whether or not the tissue section 304 on the culture medium 311 was killed; if the tissue section 304 was killed (or if the base sequence of the information tag was not taken up into the biogenic DNA of the object for introduction), that is, if the introduction of the information tag into the cell failed, then the process returns to Step S105 so that steps after Step S105 are repeated.

In Step S108, the creator determines that the information tag was introduced into the cell successfully if the cell was not killed. Then, the process proceeds to Step S109.

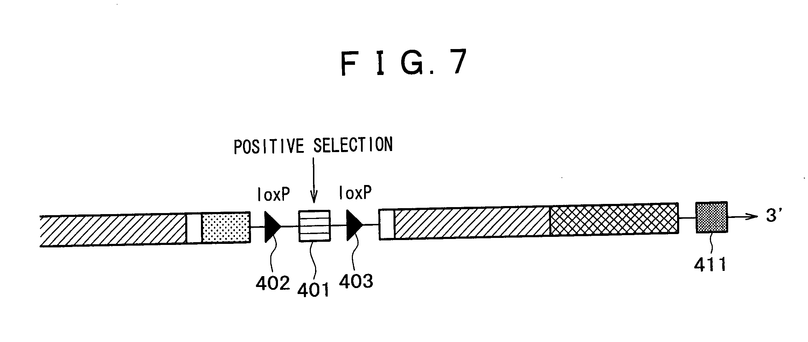

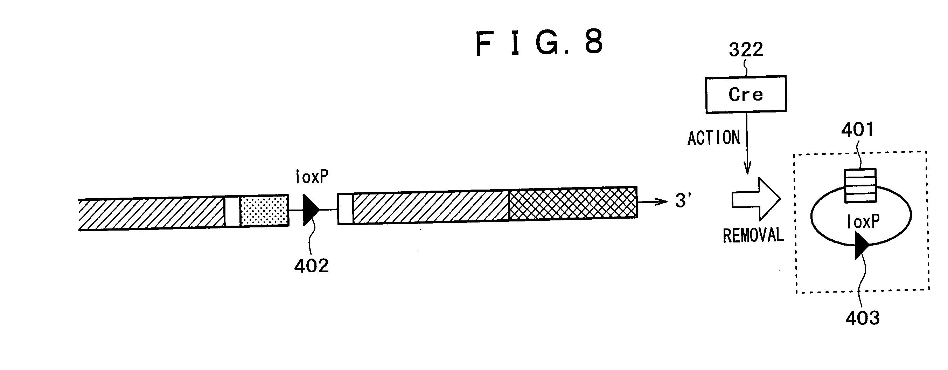

In Step S109, the creator 11 transfers the tissue section 304 which survived in the Step S108, from the culture medium 311 which is dosed with a drug as shown in FIG. 5 into the ordinary culture medium 321 which is not dosed with a drug. Then, with the aid of Cre protein 322, the drug tolerant gene 401 (shown in FIG. 7) is removed from the synthesized DNA, which has been introduced into the biogenic DNA of the tissue section 304. FIG. 8 shows how the drug tolerant gene 401 is removed. Cre protein 322 causes the drug tolerant gene 401 and loxP 403 to be removed from the biogenic DNA. Incidentally, loxP 402 remains in the biogenic DNA.

In Step S110, the creator 11 transfers the tissue section (from which the drug tolerant gene 401 for positive selection has been removed in Step S109) into the flask 331 shown in FIG. 5 and continues to culture the callus. A new individual 332 grows from the tissue section shown in FIG. 5.

The creator 11 checks the grown individual 332 for its quality (for example, to see if the gene which has been introduced in the individual 332 by gene manipulation expresses), and ships the individual 332 to the client 2. Incidentally, the individual 332 shown in FIG. 5 is identical with the information tag introduced organism 14 shown in FIG. 1.

After the foregoing steps, the process for introducing an information tag is completed.

The user 31 of the client 2 utilizes the individual 332 (which is the information tag introduced organism 14) purchased from the maker 1, in order to develop foods and drugs, for example. Moreover, if the user 31 wants to know what gene manipulation has been performed on the information tag introduced organism 14, he carries out the process for acquiring the information tag from the DNA of the organism 14, thereby reading out the information introduced.

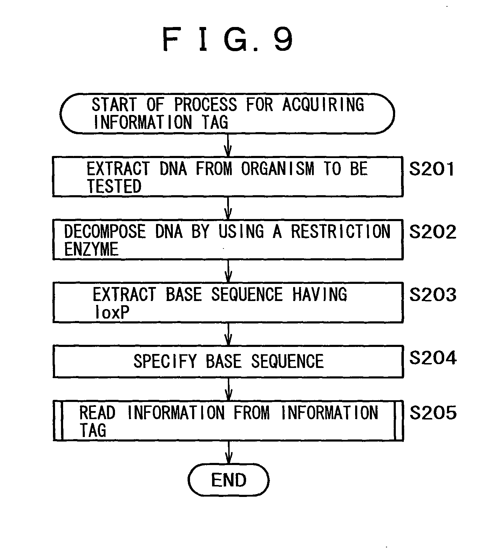

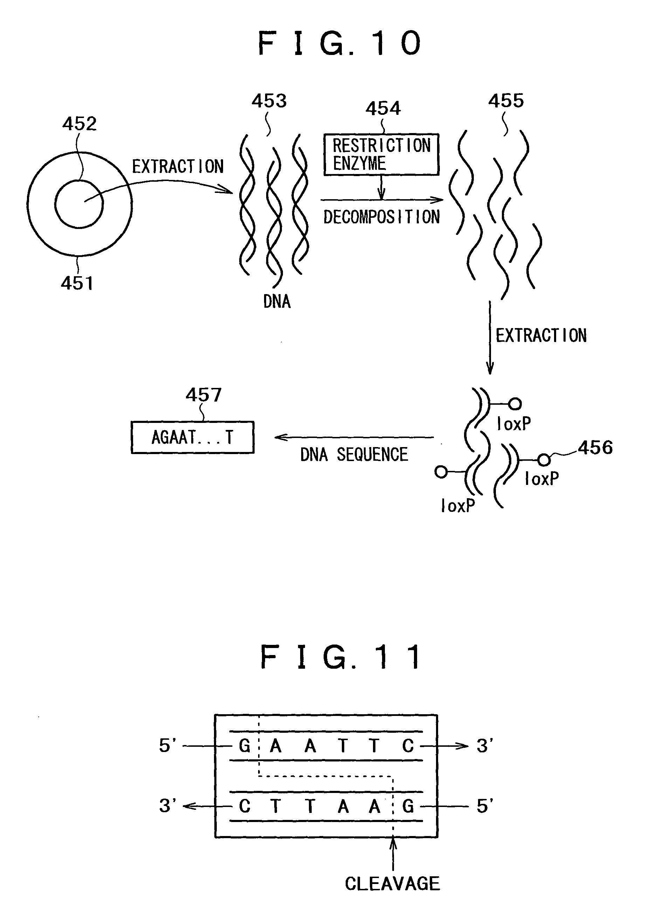

The following is concerned with the process for acquiring the information tag which is carried out by the client 2. The flowchart shown in FIG. 9 should be referenced together with FIGS. 10 and 11.

In Step S201 shown in FIG. 9, the user extracts the DNA 453 from the nucleus 452 of the cell 451 of the individual 332 which is the information tag introduced organism 14 shown in FIG. 10.

In Step S202, the user 31 cleaves the DNA which has been extracted in Step S201 at a specific position of the base sequence by using the restriction enzyme 454 shown in FIG. 10. FIG. 11 shows an example of the base sequence for cleavage by the restriction enzyme 454. It also shows the base sequence to be recognized by EcoRI (one species of restriction enzymes) and the site of cleavage. The restriction enzyme recognizes the sequence of GAATTC and cleaves the double DNA at the site between G and A. The upper part of FIG. 11 shows a base sequence GAATTC arranged from left to right or from 5'-terminus to 3'-terminus, and the lower part of FIG. 11 shows a base sequence GAATTC arranged from right to left or from 3'-terminus to 5'-terminus. The restriction enzyme 454 cleaves the base sequence at the site between adenine and guanine. As a result, the DNA 453 is cleaved into DNA fragments 455 as shown in FIG. 10. Incidentally, the base sequence having the recognition site for the restriction enzyme (shown in FIG. 11) may be arranged before and after the base sequence to be introduced, so that it is possible to decompose the DNA with the restriction enzyme and take out smoothly the desired base sequence.

In Step S203, the user 31 hybridizes the DNA fragments 455 with the base having the base sequence complementary to the loxP sequence, to which magnetic beads 456 have been bonded, as shown in FIG. 10. Then, the user 31 extracts the DNA fragments containing loxP by using a magnet. In other words, the magnetic beads 456 are attracted to the magnet. The base sequence complementary to the loxP sequence to which the magnetic beads 456 have been bonded contains the loxP sequence bonding thereto. Also, the loxP sequence bonds to the information tag as mentioned later. Therefore, by attracting the magnetic beads 456 by means of a magnet, it is possible to attract the base sequence complementary to the loxP sequence bonding to the magnetic beads 456. Moreover, it is also possible to attract the loxP sequence bonding to the base sequence complementary to the loxP sequence and the information tag bonding to the loxP sequence. In this way the DNA fragments containing loxP are extracted.

In Step S204, the user 31 introduces the DNA fragments (extracted in Step S203) into the sequencer 32. The sequencer 32 specifies the base sequence 457 of the thus introduced DNA fragments, as shown in FIG. 10. Then, the sequencer 32 supplies the base sequence data 457 (which has been specified as mentioned above) to the information acquiring apparatus 33.

In Step S205, the information acquiring apparatus 33 reads out the introduced information from the base sequence data 457 supplied from the sequencer 32, and then it displays the introduced information on the display unit 207. In this way the user 31 is able to know the information which has been introduced into the information tag introduced organism 14.

A description is made below, with reference to FIGS. 12 to 19, of the position in biogenic DNA at which the information tag is inserted and the structure of the introduced base sequence 301 containing the information tag.

FIG. 12 shows an example of the position for introduction of the base sequence in the case where the information tag is introduced into the intron region. The upper part of FIG. 12 shows an example of the structure of DNA 501, and the lower part of FIG. 12 shows an example of the structure of matured mRNA. Incidentally, FIG. 12 shows an example of the eukaryotic DNA.

The eukaryotic genes 511 are scattered on the DNA 501. There is the nongenomic region 512 which is a base sequence whose function is unknown between the genes. Each gene is composed of the control region 521 and the transcription region 522. The control region 521 includes the enhancer region 531 which controls the transcribing activity of the gene and the transcription factor recognition site (promoter region) 532. There may be an instance where the enhancer region 531 is in the intron 542-1 and 542-2 or downstream of the gene. On the other hand, the transcription region 522 includes the exon regions 541-1, 541-2, and 541-3 and the intron regions 542-1 and 542-2.

As the gene 511 is transcribed to mRNA, splicing takes place, with the result that the intron regions 542-1 and 542-2 drop out and the exon regions 541-1, 541-2, and 541-3 bind together. In the case of the matured mRNA shown in the lower part of FIG. 12, there are three exon regions 541-1, 541-2, and 541-3 and they bind together. In actual, however, there may be an instance in which selective splicing takes place and the number of exon regions eventually remaining in the matured mRNA decreases. Incidentally, the matured mRNA has the cap structure 551 and the poly A sequence 557 added thereto.

In the upstream region of the matured mRNA are the untranslated region 552 (before the translation start sequence "AUG") and the untranslated region 556 (after the translation completion sequence "UAG"). The translated region 554 held between them is translated into protein. However, the translation completion sequence may vary from one species of organism to another.

In any case, the intron region drops off from the matured mRNA and hence introducing the base sequence into this part is considered to be less influential. On the other hand, the untranslated regions 552 and 556, in which translation within mRNA is not performed, are likely to control translation and hence they are considered to affect the living organism due to change in the base sequence. Also, introducing the base sequence into the nongenomic region 512 may weaken the relation with the gene which has undergone gene manipulation. Therefore, in this embodiment, the base sequence is introduced into the intron region (e.g., the intron region 542-2) in the gene 511.

Incidentally, the foregoing does not necessarily mean that the present invention is limited to an embodiment in which the base sequence is not inserted into the untranslated region and the nongenomic region. Instead, the present invention may be practiced, with the above-mentioned defects taken into account, in such a way that the information tag is introduced into the nongenomic region and the region of the DNA 501 which is complementary to the untranslated regions 552 and 556.

FIG. 13 shows the modular structure of the base sequence to be introduced. In this example, the base sequence containing the information tag is inserted into the intron region 542-2 which is at the rearmost end of the gene shown in FIG. 12. It is also possible to insert the information tag into another intron region (e.g., the intron region 542-1 shown in FIG. 12), as a matter of course.

The upper part of FIG. 13 shows in detail the sequence of the intron region 542-2, and the lower part of FIG. 13 shows the base sequence to be introduced into the cell with the help of a vector. The sequence of the intron region usually starts with GT (e.g., GT 601 shown in FIG. 13) and ends with AG (e.g., AG 604 shown in FIG. 13). There is A (e.g., A 603 shown in FIG. 13) which is held between the recognition site at which the intron region starts and the recognition site (T-rich sequence) at which the intron region ends. This structure varies depending on the species of organisms. There is a region 602 (shown in FIG. 13) which is considered to have a loose relation with the splicing of the intron region, and it is this region 602 that the base sequence 632 containing the information tag is inserted. The region 602 is five bases downstream from the start (e.g., GT 601 shown in FIG. 13) and is five bases upstream from A (e.g., A 603 shown in FIG. 13) which is immediately before the T-rich region preceding AG (e.g., AG 604 shown in FIG. 13). Inserting an information tag into the region which is considered to have a loose relation with the intron region minimizes the influence on living organisms exerted by introduction of an information tag.

As shown in FIG. 13, the homologous gene recombination is accomplished in the following manner. A base sequence 631 is added to the upstream side of the base sequence 632 to be introduced. This base sequence 631 has the same base sequence 631 as that at the upstream side of the target region 602. This base sequence also has the same base sequence 633 as that at the downstream side of the target region 602. Moreover, the lethal gene 634 for negative selection is added to the downstream side of the base sequence 633.

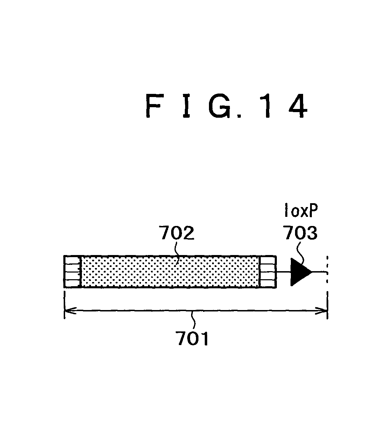

FIG. 14 shows the length of the gene to be introduced. The data base sequence 701 which has been prepared is composed of the data section 702 coded with information to be introduced and the loxP 703. The data base sequence 701 is a unit for introduction.

FIG. 15 shows in detail the structure of the base sequence 632 shown in FIG. 13.

As shown in the upper part of FIG. 15, the base sequence 632 is composed of the region 712 containing the base sequence coded with information to be introduced, the region 711 at the upstream side of the region 712, and the region 713 at the downstream side of the region 712. The base sequence 711 (at the upstream side of the region 712) and the base sequence 713 (at the downstream side of the region 712) have the same base sequence as those at the positions corresponding to the region 602 for introduction. The lower part of FIG. 15 shows in detail the structure of the region 712. Specifically, the region 712 is composed of the base sequence 721 as a whole. The region 722 (which is composed of the drug tolerant gene 723 for negative selection and the loxP 724) is removed by the Cre protein 322 after the execution of positive selection. Eventually, there remains the data base sequence 701, which is composed of the data section 702 coded with information to be introduced and the loxP 703.

Incidentally, an embodiment in which an information tag is introduced into the intron region has been explained above with reference to FIGS. 12 to 15. Now, another embodiment in which an information is introduced into a gene having no intron region will be described below. FIG. 16 shows the place at which the base sequence is introduced into a gene consisting of one exon.

The upper part of FIG. 16 shows an example of the structure of DNA 801, and the lower part of FIG. 16 shows an example of the structure of matured mRNA. In FIG. 16, the gene is composed of the control region 811 and the transcription region 812. The control region 811 includes the enhancer region 821 (which controls the transcribing activity of the gene) and the transcription factor recognition site (promoter region) 822. There may be an instance where the enhancer region 821 is at the downstream position of the gene. On the other hand, the transcription region 812 has one exon region 831.

In the case where the target gene has only one exon region 831 as shown in FIG. 16, the base sequence 833 has no direct coding for protein. The base sequence 833 is transcribed to the untranslated region 856 which follows the translation completion position "UAG" of the matured mRNA. Therefore, the base sequence should be introduced in such a way that the base sequence 833 includes the intron region. There may be an instance where the base sequence 833 is related to translation control; however, it is possible to avoid influence on translation control if the base sequence is introduced so that the intron region is included.

FIG. 17 shows the modular structure of the base sequence to be introduced into the untranslated region 833 shown in FIG. 16.

The upper part of FIG. 17 shows the untranslated region 833 which has been divided into 833a (at the upstream side) and 833b (at the downstream side). The lower part of FIG. 17 shows the base sequence to be introduced in the cell with the help of a vector. As mentioned above, the sequence of the intron region usually starts with GT and ends with AG. There is the recognition site at which the intron region starts (this recognition site is five bases from the start GT). There is also the recognition site at which the intron region ends (this recognition site is five bases upstream from A which is the head of the T-rich sequence upstream from the end AG). The region between these two recognition sites is considered to be highly related to the splicing of the intron region. Consequently, the base sequence 902 to be introduced is constructed as shown in FIG. 17. That is, at the head of the base sequence 902 is arranged the recognition site for start of the intron region. This recognition site consists of five bases counted from GT 911. At the end of the base sequence 902 is arranged the recognition site for end of the intron region. This recognition site consists of A 912, a sequence of five bases before A 912, a T-rich sequence after A, and AG 913. Between these intron regions is arranged the base sequence 921 coded with the information to be introduced. Also, for homologous gene recombination, the base sequence 901 is added to the upstream side of the base sequence 902 and the base sequence 903 is added to the downstream side of the base sequence 902. The base sequence 901 is the same one as 833a at the upstream side of the untranslated region 833 to become the target. The base sequence 903 is the same one as 833b at the down stream side of the untranslated region 833 to become the target. Also, to the downstream side of the base sequence 903 is added the lethal gene 904 for negative selection.

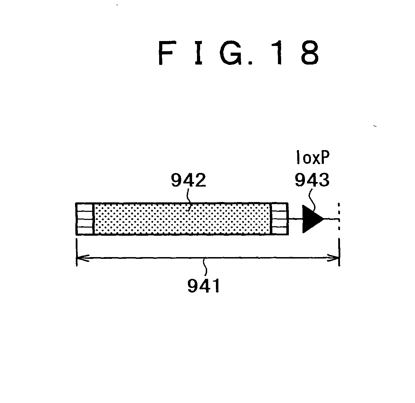

FIG. 18 shows the length of the gene to be introduced into DNA 801 shown in FIG. 16. The data base sequence 941 which has been prepared is composed of the data section 942 coded with information to be introduced and the loxP 943. The data base sequence 941 is a unit for introduction.

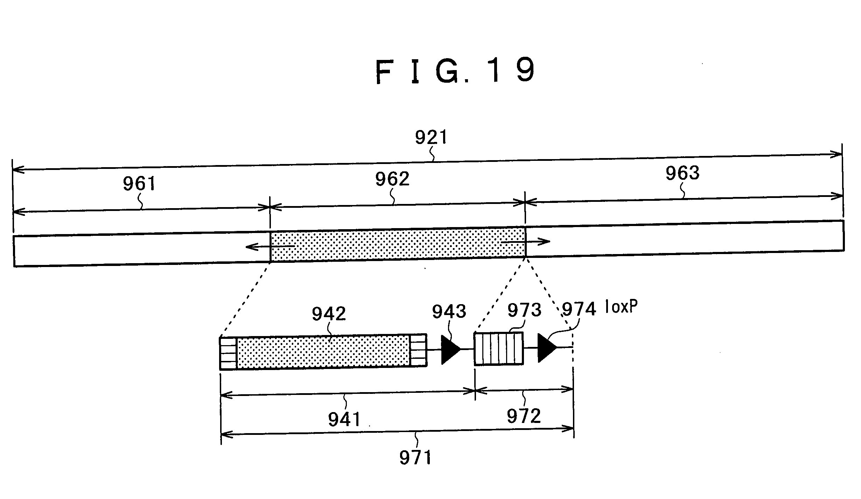

FIG. 19 shows in detail the structure of the base sequence 921 shown in FIG. 17.

As shown in the upper part of FIG. 19, the base sequence 921 is composed of the region 962 (containing the base sequence coded with information to be introduced), the region 961 (at the upstream side of the region 962), and the region 963 (at the downstream side of the region 962). The lower part of FIG. 19 shows in detail the structure of the region 962. The region 962 is initially composed of the base sequence 971 as a whole. However, the region 972 composed of the drug tolerant gene 973 for positive selection and the loxP 974 is removed by the Cre protein 322 after the execution of positive selection. Eventually, there remains the data base sequence 941, which is composed of the data section 942 coded with information to be introduced and the loxP 943.

A description is made below with reference to FIG. 20 of the process for designing the base sequence to be introduced in Step S102 in the flowchart shown in FIG. 4.

The creator 11 operates the input unit 102, thereby entering into the sequence designing apparatus 12 the information to be introduced. In Step S301, the data generating unit 103 of the sequence designing apparatus 12 generates the data for information to be introduced according to The operating information supplied from the input unit 102. FIG. 21 shows an example of the thus generated data for information to be introduced.

The data shown in FIG. 21 represent the following.
Product name 1001 is "rose".
Affiliation 1002 is "Sany".
Information about function 1003 is "rose like petal".
Information originating from gene 1004 is "native".
Information about modification of gene function 1005 is "activate".
Information about gene group 1006 is "1/12" (which
implies the first one out of 12 genes).

"Native" as the information originating from gene 1004 means that the gene itself is an existing one. The information originating from gene 1004 may be "native/modify", which means that the sequence of an existing gene has been modified. Further, the information originating from gene 1004 may be a name of the species of the organism from which the gene has been introduced. "Activate" as the information about modification of gene function 1005 means "increase". In addition, the information about modification of gene function 1005 may also be "inhibit" (which means "decrease"), "destroy" (which means "destroy"), and "insert" (which means "introduction").

The data generating unit 103 generates the data for information to be introduced according to the operating information supplied from the input unit 102, and displays the data on the display unit 104. The creator 11 can enter and modify the information to be introduced while watching the information displayed on the display unit 104. After the entry of the information to be introduced is completed, the creator 11 operates the input unit 102 and confirms the information to be introduced. After receiving from the input unit 102 the operating information indicating that the information to be introduced has been confirmed, the data generating unit 103 supplies the data compressing unit 105 with the thus generated data for information to be introduced. Then, the process proceeds to Step S302.

In Step S302, the data compressing unit 105 compresses the data supplied from the data generating unit 103 according to the previously established coding system, thereby decreasing the amount of data. Then, the data compressing unit 105 supplies the data converting unit 106 with the compressed data generated by compressing the data for information to be introduced.

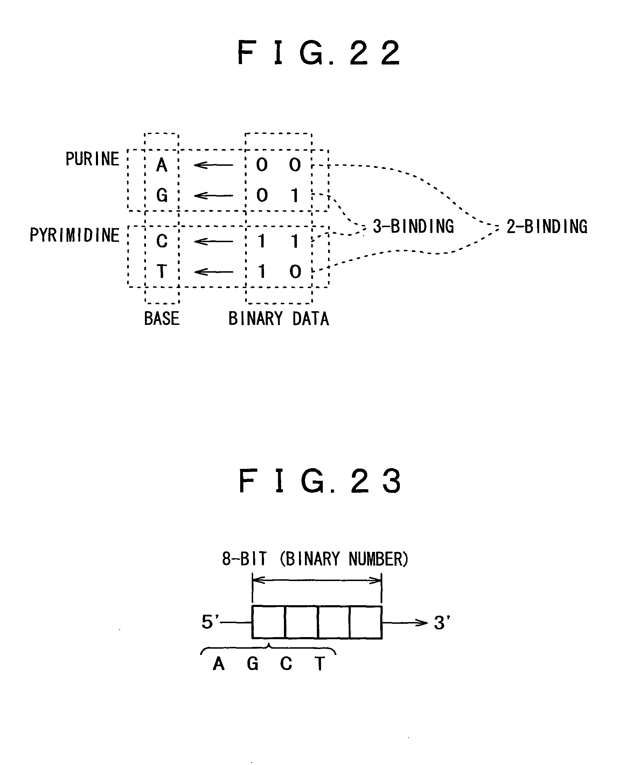

In Step S303, the data converting unit 106 converts the compressed data (as binary data) into the base sequence data. In other words, the data converting unit 106 divides the binary data into two-digit sections which correspond to the base sequence. In this way it converts the compressed data into the base sequence data. For example, the data converting unit 106 converts the compressed data into the base sequence data according to the correspondence table shown in FIG. 22.

FIG. 22 shows the correspondence between the 2-bit binary information and the base. In the example shown in FIG. 22, bases corresponding to binary data are specified in the following manner. If the first digit of the binary data is 0, then the purine base is selected. If the first digit of the binary data is 1, then the pyrimidine base is selected. If the second digit of the binary data is 1, then the 2-hydrogen bonding is selected. If the second digit of the binary data is 0, then the 3-hydrogen bonding is selected. The terms "2-hydrogen bonding" and "3-hydrogen bonding" denote the number of the hydrogen bondings which are formed between complementary bases when DNA constitutes the double helix structure.

Therefore, the data converting unit 106 converts two digits of binary data into the base as follows. "00" into A (adenine), "01" into G (guanine), "11" into C (cytosine), and "10" into T (thymine). In this way a piece of binary 8-bit data (for example) is converted into a base sequence consisting of four bases, as shown in FIG. 23.

In other words, it is possible to represent 8-bit binary information with four bases, as shown in FIG. 23. It is also possible to represent quaternary numbers with base sequence information in which one base corresponds to one bit. Expressing information in terms of binary number on a computer makes it possible to represent 8-bit information with four bases. It follows that any ASCII character can be represented by a sequence consisting of four bases. In this embodiment, a coding scheme is used that makes the four bases (AGCT) correspond to 00, 01, 11, and 10, respectively, based on their characteristic properties. However, another coding scheme may be used that represents the four bases in terms of arbitrary 2-bit data irrespective of their characteristic properties. In this case, it is necessary to properly modify the error-correcting method.

The data converting unit 106 divides the compressed binary data into 2-digit sections, thereby performing conversion into corresponding bases. In this way it generates the base sequence data and supplies it to the correction code inserting unit 107.

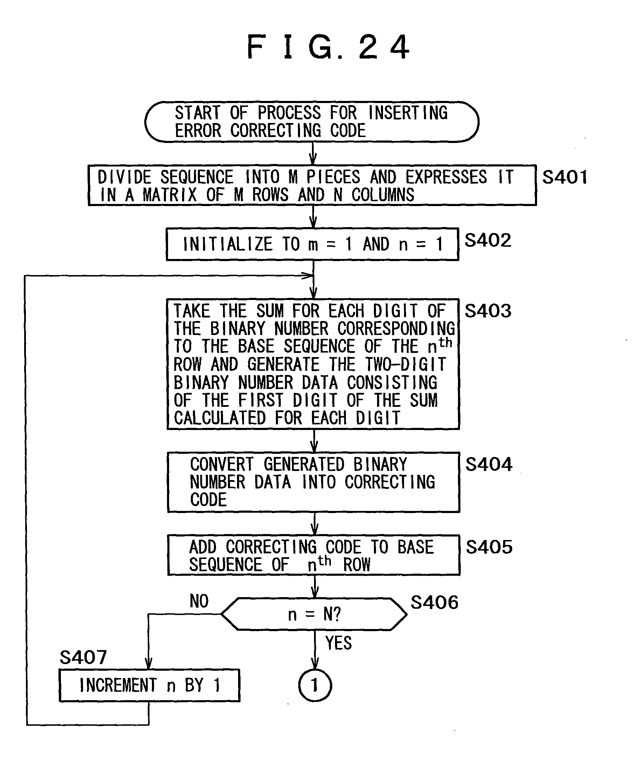

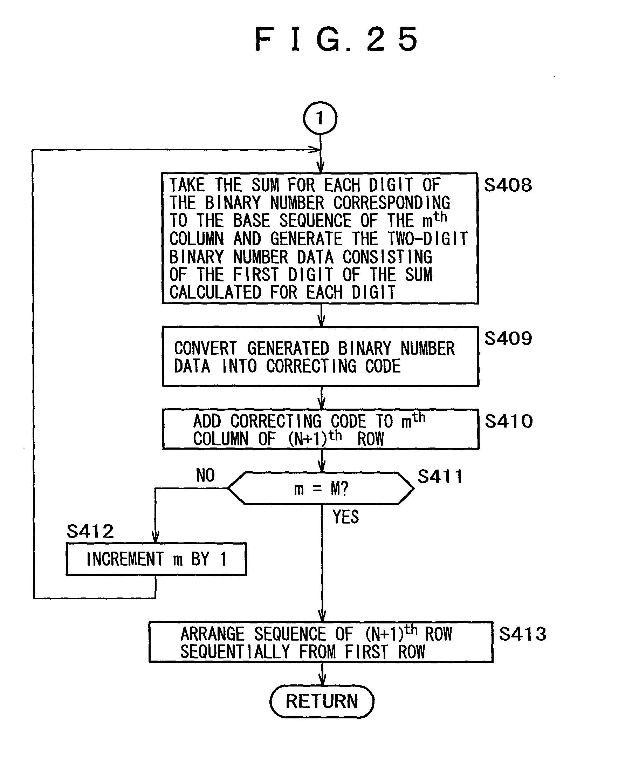

In Step S304, the correction code inserting unit 107 inserts an error correction code into the base sequence data supplied from the data converting unit 106 and then supplies the base sequence data having an error correcting data inserted thereinto to the buffer sequence inserting unit 108. Incidentally, a detailed description of Step S304 will be made later with reference to the flowcharts shown in FIGS. 24 and 25.

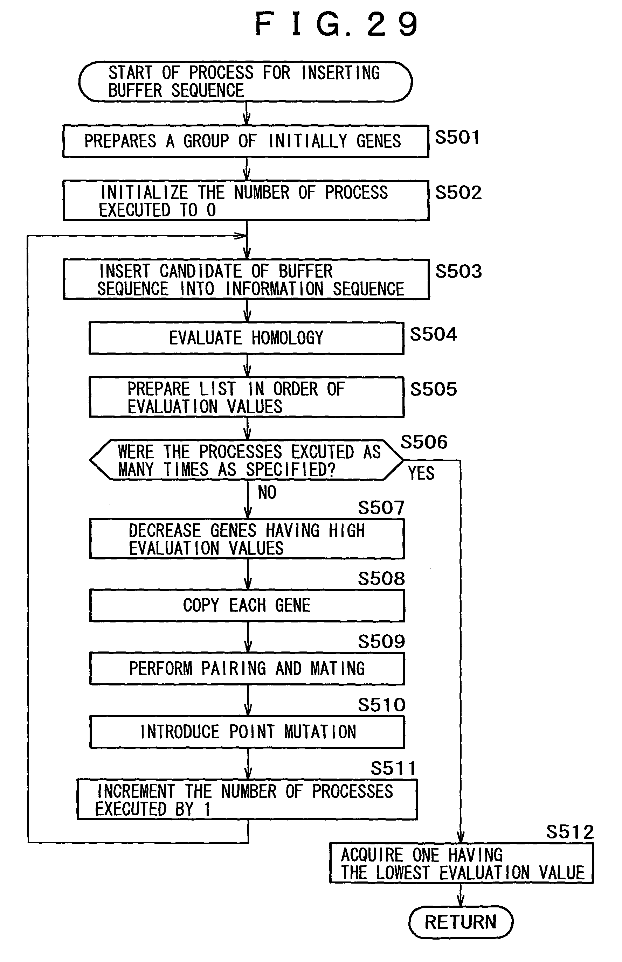

In Step S305, the buffer sequence inserting unit 108 inserts a buffer sequence to the base sequence data supplied from the correcting code inserting unit 107. In other words, if the base sequence specified by the base sequence data supplied from the error code inserting unit 107 strongly resembles by any chance the base sequence having a certain function in a living organism, there is the possibility that the base sequence introduced into the living organism disturbs the physiological function in the living organism. For this reason, the buffer sequence is inserted into the base sequence data supplied from the correcting code inserting unit 107, so that the base sequence inserted into the living organism does not resemble the base sequence which is originally present in the living organism. After inserting the buffer sequence into the base sequence data supplied from the correction code inserting unit 107, the buffer inserting unit 108 supplies the base sequence data having the buffer sequence inserted thereinto (such as data section 702 shown in FIG. 14 and data section 942 shown in FIG. 18) to the positive-selecting sequence adding unit 109. Incidentally, a detailed description of Step S305 will be given later with reference to the flowchart shown in FIG. 29.

In Step S306, the positive-selecting sequence adding unit 109 adds sequentially those base sequences corresponding to the following to the downstream side of the base sequence data supplied from the buffer sequence inserting unit 108.

One corresponding to loxP (such as loxP 703 and loxP 943 shown respectively in FIGS. 15 and 19) One corresponding to the drug-tolerant gene for positive selection (such as the drug-tolerant genes 723 and 973 for positive selection shown in respectively in FIGS. 15 and 19)

One corresponding to loxP (such as loxP 724 and loxP 974 shown respectively in FIGS. 15 and 19) Incidentally, the positive-selecting sequence adding unit 109 further adds the base sequences 711 and 713 shown in FIG. 15 and the base sequences 961 and 963 shown in FIG. 19. Then, the positive-selecting sequence adding unit 109 supplies the intron sequence adding unit 110 with the base sequence data (such as the base sequence 632 or 921 shown respectively in FIGS. 13 or 17) which has the base sequence added thereto corresponding respectively to loxP, the drug tolerant gene, and loxP.

In Step S307, the intron sequence adding unit 110 determines whether or not the region into which the information tag is introduced is the region (e.g., the base sequence 833 shown in FIG. 16) from which the untranslated region within the exon region is transcribed. In other words, if an intron region exists in the gene into which the information tag is introduced, then it is possible to introduce the information tag into the intron region. However, if no intron region exists in the gene into which the information tag is introduced, then it is necessary to introduce the information tag into the untranslated region of the gene. In this case, the intron sequence adding unit 110 reads out from the DNA sequence storing unit 118 the base sequence information of the gene into which the information tag is introduced and then determines whether or not the intron region exists in the target gene. If no intron region exits in the target gene, then the intron sequence adding unit 110 determines that the one into which the information tag is introduce is the untranslated region within the exon region. The process proceeds to Step S308.

In Step S308, the intron sequence adding unit 110 adds the base sequence data at the site which is recognized as the intron region to the regions before and after the base sequence data (e.g., the base sequence 921 shown in FIG. 17) supplied from the positive-selecting sequence adding unit 109. (The example of the recognition site includes the recognition site of the part where the intron region starts, which consists of five bases from GT 911 shown in FIG. 17, A 912, the sequence of five bases before A 912, the T-rich sequence after A, and the recognition site of the part where the intron region ends, which consists of AG 913.) The base sequence data to which to data at the site recognized as the intron is added is supplied to the introduction position determining unit 111. Then, the process proceeds to Step S309.

In Step S307, if there exists the intron region in the target gene, the intron sequence adding unit 110 determines that the region into which the information tag is introduced is the intron region. Then, the intron sequence adding unit 110 supplies the base sequence data (which has been supplied from the positive-selecting sequence adding unit 109) as such to the introduction position determining unit 111. Then, the process skips Step S308 and proceeds to Step S309.

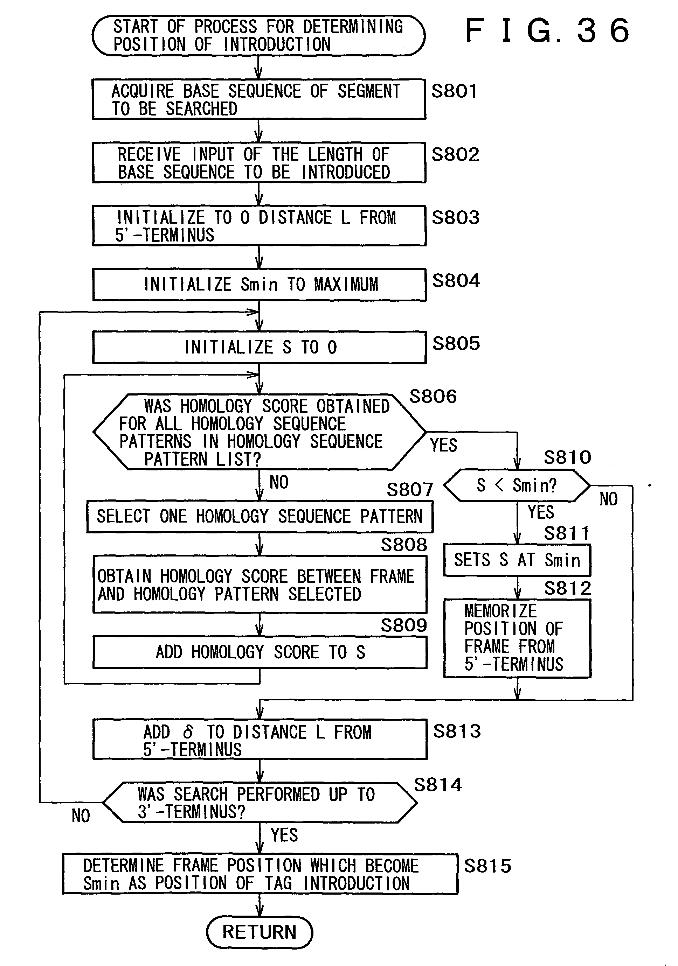

In Step S309, the introduction position determining unit 111 specifies the length of the base sequence (in terms of the number of bases) according to the base sequence data (e.g., the base sequence 632 or 902 shown respectively in FIG. 13 or 17) supplied from the intron sequence adding unit 110. Then, the introduction position determining unit 111 references the homologous sequence data, which is stored in the homologous sequence database, and the base sequence of the living organism on which gene manipulation is to be performed, which is stored in the DNA sequence storing unit 118. The introduction position determining unit 111 determines as the position into which the information tag is to be introduced the position at which insertion of the information tag is expected to have the least effect on the living organism, out of the intron (or the untranslated region) into which the information tag is to be inserted. The introduction position determining unit 111 supplies the target sequence adding unit 112 with the base sequence data supplied from the intron sequence adding unit 110 and the information showing the position at which the information tag is introduced. Incidentally, a detailed description of Step S309 will be given later with reference to the flowchart shown in FIG. 36.

In Step S310, the target sequence adding unit 112 adds the sequence that brings about homologous recombination to the base sequence data supplied from the introduction position determining unit 111. In other words, the target sequence adding unit 112 reads out the base sequence (e.g., the base sequence 631 and 633 shown in FIG. 13 and the base sequence 901 and 903 shown in FIG. 17) corresponding to the upstream and downstream sides of the position at which the information tag is introduced, out of the base sequence of the living organism on which gene manipulation is performed, which is stored in the DNA sequence storing unit 118. The base sequence is read out according to the introduction position information supplied from the introduction position determining unit 111. Then, the target sequence adding unit 112 adds the corresponding base sequence to the upstream and downstream sides of the base sequence data supplied from the introduction position determining unit 111. The target sequence adding unit 112 supplies the negative-selecting sequence adding unit 113 with the base sequence data which has added thereto the base sequence of biogenic DNA corresponding to the upstream and downstream sides of the position at which the information tag is introduced.

In Step S311, the negative-selecting sequence adding unit 113 adds the base sequence of lethal gene for negative selection (e.g., the lethal gene for negative selection 634 or 904 shown respectively in FIG. 13 or 17) to the base sequence data supplied from the target sequence adding unit 112, and supplies the result to the output unit 114. The output unit 114 supplies the DNA synthesizing apparatus 13 with the base sequence data supplied from the negative-selecting sequence adding unit 113 as the sequence design data.

Now, the process for designing the base sequence to be introduced is completed, and the process proceeds to Step S103 shown in FIG. 4.

A detailed description is made below with reference to FIGS. 24 and 25 of the process for inserting the error correction code in Step S304 shown in FIG. 20.

In Step S401 shown in FIG. 24, the correction code inserting unit 107 divides the base sequence (which has been specified by the base sequence data supplied from the data converting unit 106) into sections each consisting of a previously established number (M) of bases. The divided bases are represented by a matrix composed of N rows and M columns, as shown in FIG. 26.

In FIG. 26, the numeral 1051 indicates the first row of the base sequence which has been divided into N rows. As FIG. 26 shows, the base sequence specified by the base sequence data is subsequently divided into the second to N^{th} rows which follow the first row 1051.

In Step S402, the correction code inserting unit 107 initializes the variables m and n (which specify the row and column) to m = 1 and n = 1, respectively.

In Step S403, the correction code inserting unit 107 obtains the binary data corresponding to respective bases constituting the base sequence in the n^{th} row according to the base-binary relation shown in FIG. 22, and then sums up separately the first and second digits of the thus obtained binary data as shown in FIG. 27. In other words, in FIG. 27, the numerals 1101-1 to 1101-M denote the 2-digit binary data obtained from M bases constituting the base sequence in the n^{th} row. First, the correction code inserting unit 107 sums up the values of the second digit of the binary data 1101-1 to 1101-M. Thus, the correction code inserting unit 107 acquires the value 1151A (which is the sum of the values of the second digit and which represents the first digit of the sum). The correction code inserting unit 107 sums up the values of the first digit of the binary data 1101-1 to 1101-M. Thus, the correction code inserting unit 107 acquires the value 1151B (which is the sum of the values of the first digit and which represents the first digit of the sum).

Therefore, for example, if the n^{th} row is a base sequence "AGCTT" consisting of five bases, the correction code inserting unit 107 obtains binary data "00", "01", "11", "10", and "10" according to the corresponding relation shown in FIG. 22. Then, the correction code inserting unit 107 obtains a sum "11" (in binary number) from the values "0", "0", "1", "1", and "1" of the second digit of the binary number data and thus acquires the value "1" (which is the value of the first digit of "11") as the value of 1151A. Further, the correction code inserting unit 107 obtains a sum "10" (in binary number) from the values "0", "1", "1", "0", and "0" of the first digit of the binary number data and thus acquires the value "0" (which is the value of the first digit of "10") as the value of 1151B. The correction code inserting unit 107 obtains the 2-digit binary data "10", in which the value of 1151A is the value of the second digit and the value of 1151B is the value of the first digit.

In Step S404, the correction code inserting unit 107 converts the 2-digit binary data (consisting of the values of 1151A and 1151B) into bases according to the correspondence relation shown in FIG. 22. For example, if 2-digit binary data "10" is obtained in Step S403 as mentioned above, the correction code inserting unit 107 converts the binary data "10" into T (thymine) according to the correspondence relation shown in FIG. 22.

In Step S405, the correction code inserting unit 107 adds, as the correction code, the base data of the base converted from the binary data in Step S404 to the end of the 3'-terminus of the base sequence in the n^{th} row. In FIG. 26, the correction code 1052-1 represents the base data which has been added in Step S405 based on the base sequence data in the row 1051.

In Step S406, the correction code inserting unit 107 determines whether or not the variable n equals N (or n = N). If n does not equal N (or n < N), then the process proceeds to Step S407.

In Step S407, the correction code inserting unit 107 increments the variable n by 1. Then, the process returns to Step S403 and repeats its subsequent steps.

As the result of repeating Steps S403 to S407, the correction codes 1052-1 to 1052-N (corresponding to the first to N^{th} rows in FIG. 26) are added to the end of the 3'-terminus of each row.

After the correction codes corresponding to all the rows have been added, the correction code inserting unit 107 determines that n = N in Step S406, and the process proceeds to Step S408.

The correction code inserting unit 107 performs Steps S408 to S412 shown in FIG. 25. In these steps, it adds the correction codes 1062-1 to 1062-M to the first to M^{th} columns, in the same way as it did for rows in the foregoing steps.

In other words, in Step S408, the correction code inserting unit 107 obtains the binary data corresponding to respective bases constituting the base sequence in the m^{th} column according to the base-binary relation shown in FIG. 22, and then sums up separately the first and second digits of the thus obtained binary data in the same way as explained above with reference to FIG. 27. Thus, it acquires the 2-digit binary data consisting of the values of the first digit of each sum. In other words, operation is performed on each digit of the binary number such that 0 is assigned when the number of bases for "1" is even and 1 is assigned when the number of bases for "0" is odd, or operation is performed to obtain the remainder left after division of the sum by 2.

In Step S409, the correction code inserting unit 107 converts the 2-digit binary data (which has been obtained in Step S408) into bases according to the correspondence relation shown in FIG. 22.

In Step S410, the correction code inserting unit 107 adds, as the correction code, the base converted from the binary data in Step S409 to the m^{th} column of the base sequence in the (N+1)^{th} row. In FIG. 26, the correction code 1062-1 represents the base data which has been added in Step S410 based on the base sequence data in the column 1061.

In Step S411, the correction code inserting unit 107 determines whether or not the variable m equals M (or m = M). If m does not equal M (or m < M), then the process proceeds to Step S412.

In Step S412, the correction code inserting unit 107 increments the variable m by 1. Then, the process returns to Step S408 and repeats its subsequent steps.

As the result of repeating Steps S408 to S412, the correction codes 1062-1 to 1062-M (corresponding to the first to M^{th} columns in FIG. 26) are added to the (N+1)^{th} row.

After the correction codes corresponding to all the columns have been added, the correction code inserting unit 107 determines that m = M in Step S411, and the process proceeds to Step S413.

In Step S413, the correction code inserting unit 107 sequentially connects as follows the base sequences of the first to (N+1)^{th} rows to which were added the correction codes 1052-1 to 1052-N and the correction codes 1062-1 to 1062-M shown in FIG. 26. With the base sequence of the first row being the head, the 5'-terminus of the base sequence of the second row is connected to the 3'-terminus of the base sequence of the first row, the 5'-terminus of the base sequence of the third row is connected to the 3'-terminus of the base sequence of the second row, and so on, until the base sequence of the (N+1)^{th} row is connected. In this way there is formed the base sequence data having the correction code inserted thereinto.

The process for inserting the error correction code is accomplished as mentioned above. The effect of inserting the error correction code is that even though the bases of DNA are partly replaced after the DNA coded with information has been introduced into the biogenic DNA, it is possible to make correction. This permits one to retrieve more accurately the information which has been introduced.

A description is given below, with reference to FIG. 28, of the buffer sequence inserted by the buffer sequence inserting unit 108.

As mentioned above, there may be an instance where the base sequence specified by the base sequence data supplied from the correction code inserting unit 107 strongly resembles by any chance the base sequence (e.g., that of enhancer and promoter) having a certain function in a living organism. In such a case, there is the possibility that the base sequence introduced into the living organism disturbs the physiological function in the living organism. For this reason, the buffer sequence inserting unit 108 inserts a buffer sequence into the base sequence data supplied from the correction code inserting unit 107, so that the base sequence inserted into the living organism does not resemble the base sequence which is originally present in the living organism.

In the example shown in FIG. 28, the buffer sequence inserting unit 108 arranges the base sequence 1202-1 consisting of k bases (bit expressed in quaternary number) and then arranges one buffer sequence (spacer) 1201-1 out of the base sequence data supplied from the correction code inserting unit 107. Also, the buffer sequence inserting unit 108 arranges, after the buffer sequence (spacer) 1201-1, the base sequence 1202-2 that follows the base sequence 1202-1, out of the base sequence data supplied from the correction code inserting unit 107. Moreover, it arranges one buffer sequence (spacer) 1201-2 after the base sequence 1202-2. In this way the buffer sequence inserting unit 108 inserts the buffer sequence (spacer) for every k bases of the base sequence specified by the base sequence data supplied from the correction code inserting unit 107. Incidentally, it is permissible to arrange two or more buffer sequences side by side.

It is assumed that the arrangement of the base sequence to be inserted into the terminus of information is the sequence that recognizes the cleave site of the restriction enzyme (e.g., the recognition site of restriction enzyme as shown in FIG. 11). In this case it is possible to design such that the base sequence of the DNA to be introduced into a living organism does not become the recognition base sequence if the distance (k) for insertion of the buffer sequence is such that k < 6, with the length of the recognition site being six bases. In addition, the buffer sequence inserting unit 108 properly establishes the buffer sequence so that it does not take the base sequence similar to the base sequence which becomes other control signal, such as TATA box which is a transcription start signal.

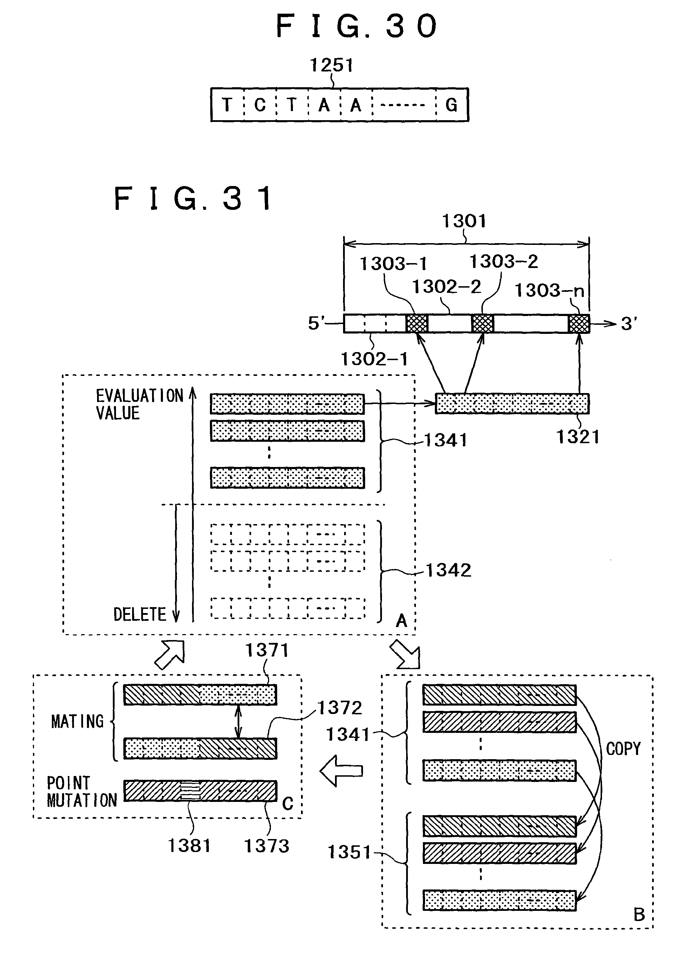

A detailed description is made below, with reference to the flowchart shown in FIG. 29, of the process of inserting the buffer sequence as Step S05 shown in FIG. 20. Meanwhile, a description is made arbitrarily with reference to FIGS. 30 and 31. Incidentally, the following description employs a genetic algorithm (GA).

In Step S501 shown in FIG. 29, the buffer sequence inserting unit 108 prepares a group of initially genes. Incidentally, the "gene" of the "initially gene" is a gene in the genetic algorithm or the candidate of the buffer sequence, but is not a gene in the biological sense. FIG. 30 illustrates an initially gene 1251 out of a group of early genes. The initially gene 1251 looks like a collection of the buffer sequences (spacers) of the base sequence to be introduced (e.g., the buffer sequences 1201-1 to 1201-n shown in FIG. 28). The buffer sequence inserting unit 108 prepares more than one initially gene (such as the initially gene 1251) consisting of a plurality of different base sequence patterns.

In Step S502, the buffer sequence inserting unit 108 initializes to 0 the number of processes executed.

In Step S503, the buffer sequence inserting unit 108 evaluates homology by utilizing the homology score stored in the homologous sequence database 117. In other words, the homologous sequence database 117 stores the base sequence pattern that appears in common with the genome of a plurality of species of organisms, such that they correspond to the homology score as an index for their importance. The buffer sequence inserting unit 108 inserts the candidate of each buffer sequence in a group of initially genes (such as the initially gene 1251) into the base sequence specified by the base sequence data. After that, in Step S504, the buffer sequence inserting unit 108 evaluates how the base sequence pattern of the base sequence data in which the buffer sequence has been inserted in Step S503 resembles the homologous sequence pattern stored in the homologous sequence database 117.

In Step S505, the buffer sequence inserting unit 108 prepares a list in the order of evaluation values according to the result of evaluation of homology in Step S504.

In Step S506, the buffer sequence inserting unit 108 determines whether or not the processes were executed as many times as previously established. If this requirement is not met, the process proceeds to Step S507.

In Step S507, the buffer sequence inserting unit 108 deletes the gene (the candidate of the buffer sequence) which has a high evaluation value (or has a great resemblance to the homologous sequence pattern).

FIG. 31 illustrates by example how to search the buffer sequence by the genetic algorithm. The process of genetic algorithm consists of three steps, that is, A: evaluation, B: copying, and C: mating and mutation. The buffer sequence inserting unit 108 repeats these steps A to C as many times as specified. Then, it takes out the gene having the lowest evaluation value (the candidate of the buffer sequence) and inserts it into the base sequence specified by the base sequence data, thereby preparing the base sequence data into which the buffer sequence is inserted.

Step S507 shown in FIG. 29 corresponds to the evaluation step A shown in FIG. 31. In this step, it evaluates homology, prepares a list showing those which have evaluation values in ascending order, and deletes the group of the buffer sequence candidates 1342 having evaluation values above the threshold value (more than the given number counted from the head of the list). This step leaves the group of the buffer sequence 1341 having small evaluation values.

In Step S508, the buffer sequence inserting unit 108 copies the remaining genes.

For example, in FIG. 31, the copying step B (corresponding to Step S508 shown in FIG. 29) copies the existing gene (the buffer sequence candidate 1341) and generates the group of the buffer sequence candidates 1351 having the same sequence as the group of the buffer sequence candidate 1341.

In Step S509, the buffer sequence inserting unit 108 makes a pair from two genes for gene mating. Also, in Step S510, the buffer sequence inserting unit 108 introduces point mutation.

For example, Steps S509 and S510 shown in FIG. 29 correspond to the mating/mutation step C shown in FIG. 31. In the step C, the buffer sequence candidates 1371 and 1372 in pair are selected for the genes in a given ratio and a portion of the sequence is exchanged. Also, according to the given ratio, the base code 1381 at one place of the buffer sequence candidate 1381 is replaced by another base code.

In Step S511, the buffer sequence inserting unit 108 increments the number of processes executed by 1. After that, the process returns to Step S503 and repeats the steps that follow Step S503. The repetition of Steps S503 to S511 reduces similarity between the base sequence of the base sequence data into which the buffer sequence has been introduced and the base sequence of the biogenic DNA.

And, in Step S506, the buffer sequence inserting unit 108 determines that the steps were repeated as many times as previously established. Then, the process proceeds to Step S512. In Step S512, the buffer sequence inserting unit 108 inserts the candidate of the buffer sequence having the lowest evaluation value out of a plurality of the candidate of the buffer sequence into the base sequence data, and acquires it as the base sequence data in which the buffer sequence is inserted.

In the way mentioned above, the process for inserting the buffer sequence is executed. By inserting the buffer sequence into the base sequence data supplied from the correction code inserting unit 107 as mentioned above, it is possible to prevent the base sequence to be introduced into a living organism from resembling the base sequence which is originally present in the living organism. As the result, it is possible to prevent the base sequence of the synthesized DNA from resembling the base sequence (such as the base sequence of enhancer and promoter) having a certain function in a living organism, thereby to prevent it from disturbing the original physiological function of the living organism.

A description is made below, with reference to the flowchart shown in FIG. 32, of the process for preparing the homologous sequence database. In this process, the homologous sequence pattern acquiring unit 116 specifies the base sequence pattern that appears in common with the genomes of a plurality of species of organisms out of the genomes of a plurality of species of organisms stored in the DNA library 115 and calculates the homology score as an index for importance of its base sequence pattern and stores it in the homologous sequence database 117.

In Step S601 shown in FIG. 32, the control unit 101 acquires the base sequence data of the genomes of a plurality of species of organisms from an external apparatus through a communication unit (not shown) and stores it in the DNA library 115. Incidentally, the base sequence data of the genomes of a plurality of species of organisms can be obtained by using, for example, the all-genome shotgun system.

In Step S602, the homologous sequence pattern acquiring unit 116 eliminates the base sequence corresponding to a promoter and the base sequence corresponding to a gene from the base sequence specified by the base sequence data of the genomes of a plurality of specifies of organisms which is stored in the DNA library 115. In other words, the homologous sequence pattern acquiring unit 116 eliminates the base sequence corresponding to a gene by using the gene estimating program that employs the hidden Markov model. The homologous sequence pattern acquiring unit 116 also excludes the promoter region which exists at the 5'-terminus of the gene sequence.

In Step S603, the homologous sequence pattern acquiring unit 116 executes the process for acquiring the homologous sequence and obtains the ratio of appearance and the average score of the sequence pattern of the base sequence which is well-preserved (or existing in common) in the genome of a plurality of species of organisms. Incidentally, a detailed description of the processing by Step S603 will be given later.

In Step S604, the homologous sequence pattern acquiring unit 116 multiplies the average score by the ratio of appearance of the sequence pattern of the base sequence obtained in Step S603, thereby to calculate the homology score.

In Step S605, the homologous sequence pattern acquiring unit 116 stores in the homologous sequence database 117 the homologous sequence pattern acquired in Step S603, by associating it with the homology score obtained in Step S604.

In the way mentioned above, the homologous sequence database 117 stores the homologous sequence pattern and its homology score. Incidentally, the homology search, which is a process corresponding to Steps S603 and S604 mentioned above, can be accomplished by BLAST (Basic Local Alignment Search Tool) as a standard tool for homology search. The homologous sequence pattern acquiring unit 116 can accomplish the homology search by the same process as BLAST.

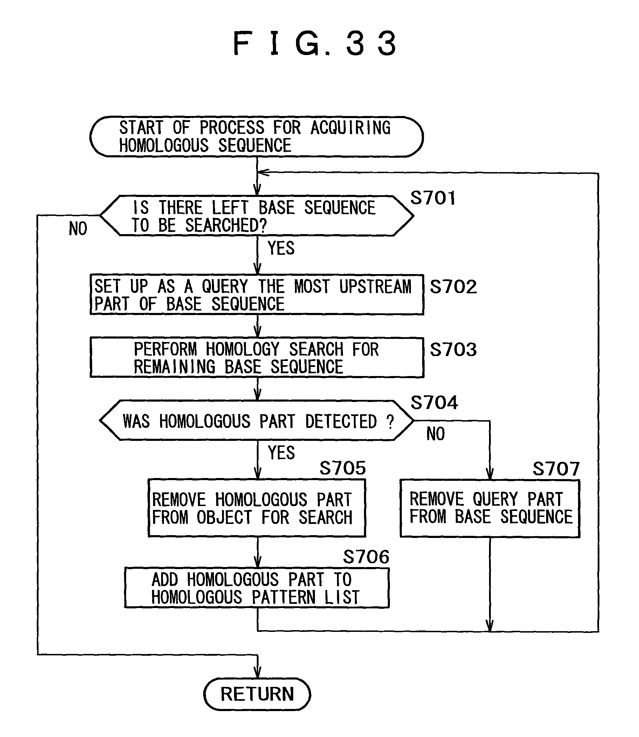

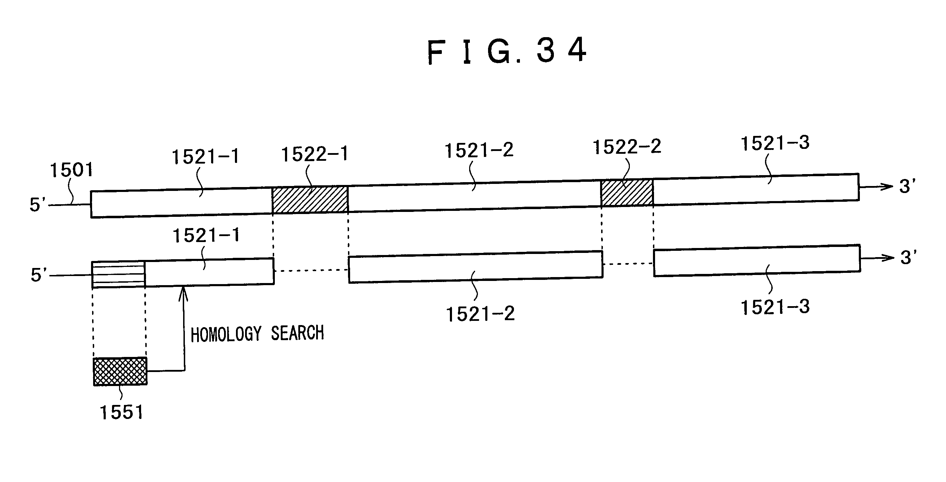

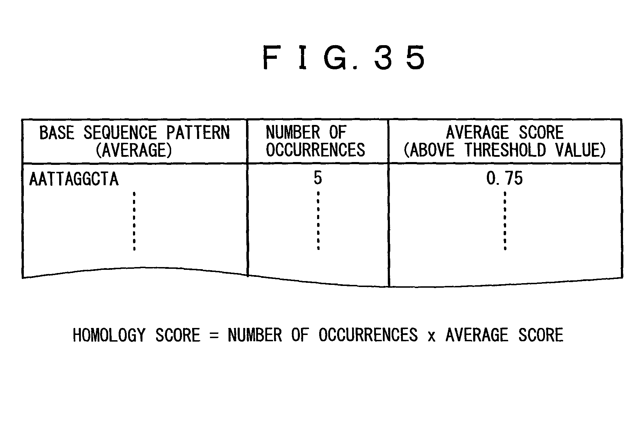

A detailed description will be made below, with reference to the flowchart shown in FIG. 33, together with FIGS. 34 and 35 occasionally, of the process for acquiring the homologous sequence in Step S603 shown in FIG. 32.

In Step S701 shown in FIG. 33, the homologous sequence pattern acquiring unit 116 determines whether or not a base sequence as the object of search for the homologous sequence pattern remains. If such a base sequence remains, the process proceeds to Step S702. Incidentally, immediately after the start of the process shown in FIG. 33, the base sequence, from which the sequence corresponding to the promoter and gene has been removed in Step S602 shown in FIG. 32, still remains. Consequently, the homologous sequence pattern acquiring unit 116 determines that the base sequence as the object of search for the homologous sequence pattern remains, and the process proceeds to Step S702.

FIG. 34 shows this state. In other words, the upper part of FIG. 34 shows an example of the base sequence specified by the base sequence data of the genome stored in the DNA library, and the lower part of FIG. 34 shows the base sequence from which the sequence corresponding to the promoter and gene has been removed. The base sequence 1501 specified by the base sequence data of the genome stored in the DNA library 115 is composed of the base sequences 1522-1 and 1522-2 (which code the promoter and gene) and the base sequences 1521-1 to 1521-3 (which do not code the promoter and gene). Of these base sequences, the base sequences 1522-1 and 1522-2 (which code the promoter and gene) are removed, as shown in the lower part of FIG. 34, in Step S602 shown in FIG. 32. The homologous sequence pattern acquiring unit 116 determines whether or not search for the homologous pattern has been performed on all of the base sequences 1521-1 to 1521-3. In this way it determines whether or not the base sequence as the object for search remains. In other words, if any base sequence which has the homologous sequence pattern not searched yet remains in the base sequences 1521-1 to 1521-3, the homologous sequence pattern acquiring unit 116 determines that the base sequence as the object for search remains. If search for the homologous sequence pattern has been performed on all of the base sequences 1521-1 to 1521-3, the homologous sequence pattern acquiring unit 116 determines that the base sequence as the object for search does not remain.

In Step S702, the homologous sequence pattern acquiring unit 116 sets up as a query the base sequence (as many as specified) at the most upstream side out of the base sequences which have not yet been searched. In other words, the homologous sequence pattern acquiring unit 116 takes out the base sequence in the striped region in the lower left part of FIG. 34 and sets up it as the query 1551.

In Step S703, the homologous sequence pattern acquiring unit 116 performs homology search between the base sequence pattern of the query 1551 and the remainder of the base sequence remaining as the object for search. In other words, the homologous sequence pattern acquiring unit 116 calculates homology between the base sequence pattern of the query 1551 and the base sequence pattern of the base sequence remaining as the object for search at the downstream side from the query 1551, then it slides the base sequence pattern of the query 1551 along the base sequence remaining as the object for search in the downstream side from the query 1551.

In Step S704, the homologous sequence pattern acquiring unit 116 determines whether or not any homologous part with homology higher than a standard value has been detected between the base sequence pattern of the query 1551 and the remainder of the base sequence remaining as the object for search. If any homologous part with homology higher than a standard value is detected between the base sequence pattern of the query 1551 and the remainder of the base sequence remaining as the object for search, then the process proceeds to Step S705.

In Step S705, the homologous sequence pattern acquiring unit 116 removes the detected homologous part from the base sequence as the object for homology search. At this time, the homologous sequence pattern acquiring unit 116 also removes the base sequence corresponding to the query 1551 from the base sequence as the object for search.

In Step S706, the homologous sequence pattern acquiring unit 116 adds the base sequence pattern of the detected homologous part to the list of the homologous sequence pattern. FIG. 35 shows an example of the homologous sequence pattern. In other words, the list of the homologous sequence pattern associates with one another the base sequence pattern as the homologous sequence pattern, the frequency of occurrence of the homologous sequence pattern, and the average score of the homologous sequence pattern. After Step S706, the process returns to Step S701 and repeats the steps that follow Step S701 mentioned above.

In Step S704, the homologous sequence pattern acquiring unit 116 may determine that any homologous part whose homology is higher than the standard value is not detected between the base sequence pattern of the query 1551 and the remainder of the base sequence remaining as the object for search. In this case, the process proceeds to Step S707.

In Step S707, the homologous sequence pattern acquiring unit 116 removes the base sequence corresponding to the query 1551 from the base sequence as the object for search. After that, the process returns to Step S701 and repeats the steps that follow Step S701 mentioned above.

In Step S701, the homologous sequence pattern acquiring unit 116 may determine that any base sequence as the object for search does not remain any more (or search has been completed for all the base sequences). In this case, the process for acquiring the homologous sequence terminates and the process proceeds to Step S604 shown in FIG. 32.

In the way mentioned above, the process for acquiring the homologous sequence is executed. The foregoing process acquires a plurality of base sequence patterns having a score higher than the threshold value.

A detailed description will be made, with reference to the flowchart shown in FIG. 36, of the process for determining the position of introduction, as Step S309 shown in FIG. 20.

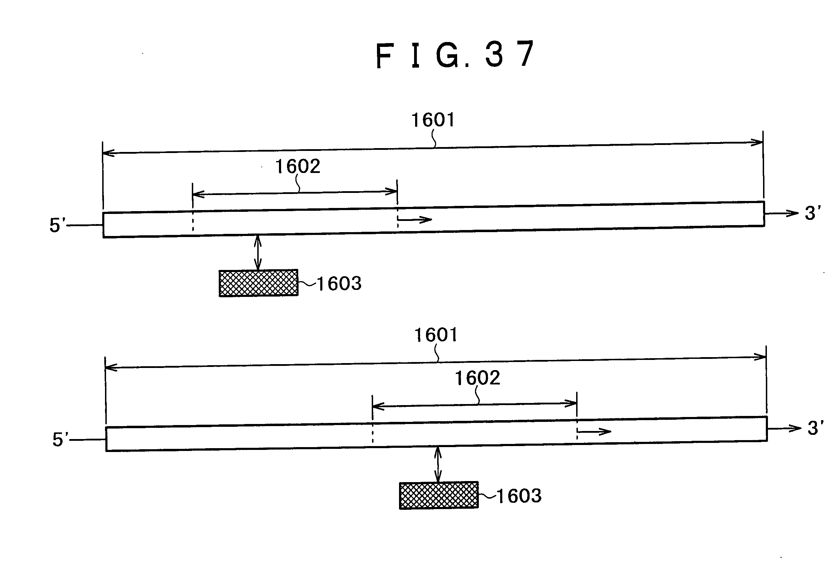

In Step S801, the introduction position determining unit 111 acquires from the DNA sequence storing unit 118 a part of the base sequence of the intron region into which the information tag is to be introduced (or the complementary sequence corresponding to the untranslated region), as the section for search. Incidentally, a part of the base sequence of the intron region is exemplified by the section from the downstream position (five bases of GT 601) to the upstream position (five bases of A603), both shown in FIG. 13. Also, the complementary sequence corresponding to the untranslated region is exemplified by the section of the base sequence 833 shown in FIG. 16. The section indicated by the code 1601 in FIG. 37 is the section for search.

In Step S802, the introduction position determining unit 111 acquires the base sequence length of the base sequence to be introduced (e.g., the data base sequence 701 shown in FIG. 14 or the data base sequence 941 shown in FIG. 18). The length of the section indicated by the code 1602 in FIG. 37 is the base sequence length of the base sequence to be introduced.

In Step S803, the introduction position determining unit 111 initializes the length L (from the 5'-terminus to be searched) to 0. In Step S804, the introduction position determining unit 111 initializes the minimum value (Smin) of the homology score to the actual maximum value which the accumulating homology score S substantially takes on. In Step S805, the introduction position determining unit 111 initializes to 0 the accumulating homology score S at a position of interest.

In Step S806, the introduction position determining unit 111 determines whether or not the homology score has been obtained between the base sequence pattern at the position of the distance L away from the 5'-terminus and all the homologous sequence patterns of the homologous sequence pattern list stored in the homologous sequence database 117. If it determines that there is any homologous sequence pattern which has not yet obtained the homology score between the base sequence pattern at the position of the distance L away from the 5'-terminus and the homologous sequence patterns of the homologous sequence pattern list stored in the homologous sequence database 117, then the process proceeds to Step S807.

In Step S807, the introduction position determining unit 111 acquires one homologous sequence pattern for which the homology score has not yet been calculated, from the homologous sequence pattern list stored in the homologous sequence database 117. In Step 808, the introduction position determining unit 111 calculates the homology score between the homologous sequence pattern acquired in Step S807 and the sequence pattern of the base sequence at the position of the distance L away from the 5'-terminus. In Step S809, the introduction position determining unit 111 adds the homology score calculated in Step S808 to the accumulating homology score S. After that, the process returns to Step S806 and repeats the steps that follow Step S806 mentioned above.

What is done in Steps S806 to S809 is to evaluate the homology between the frame 1602 established at the position in the section 1601 for search and all the homologous sequence patterns contained in the homologous sequence pattern list. (See the upper part of FIG. 37.)

In Step S806, the introduction position determining unit 111 may determine that the homology score has been obtained between the base sequence pattern at the position the length L away from the 5'-terminus and all the homologous sequence patterns of the homologous sequence pattern list stored in the homologous sequence database 117. In this case, the process proceeds to Step S810.

In Step S810, the introduction position determining unit 111 determines whether or not the accumulating homology score S is smaller than the minimum value Smin. If the accumulating homology score S is smaller than the minimum value Smin, then the process proceeds to Step S811. In Step S811, the introduction position determining unit 111 substitutes the accumulating homology score S for Smin. As a result, the minimum value Smin becomes a further smaller value. In Step S812, the introduction position determining unit 111 memorizes the currently established distance L from the 5'-terminus as the distance Lmin for the minimum value Smin. After that, the process proceeds to Step S813.

In Step S810, the introduction position determining unit 111 may determine that the accumulating homology score S is not smaller than the minimum value Smin (the accumulating homology score S is larger than the minimum value Smin). In this case, the Steps S811 and S812 are skipped, and the process proceeds to Step S813.

In Step S813, the introduction position determining unit 111 adds the previously established base number δ to the distance L from the 5'-terminus, thereby renewing the distance L.

In Step S814, the introduction position determining unit 111 determines whether or not the position of the distance L away from the 5'-terminus is the 3'-terminus, thereby determining whether or not all searches up to the 3'-terminus have been executed. If the position of the distance L away from the 5'-terminus is not the 3'-terminus, it determines that searches up to the 3'-terminus have not yet been executed. And, the process returns to Step S805 and repeats the steps that follow Steps S805 mentioned above.

As the result, the position of the frame 1602 is shifted toward the 3'-terminus as shown in the lower part of FIG. 37, for example, and homology at that position is evaluated. The procedures in Steps S806 to S814 are repeated, and the position Lmin is obtained at which the accumulating homology score is minimum at a plurality of positions in the search section 1601.

In Step S814, the introduction position determining unit 111 determines that all searches up to the 3'-terminus have been accomplished if the position of the distance L away from the 5'-terminus is the 3'-terminus. And the process proceeds to Step S815.

In Step S815, the introduction position determining unit 111 determines as the position at which the information tag is to be introduced the distance Lmin from the 5'-terminus when it takes on the minimum value Smin.

The process for determining the introduction position is executed as mentioned above. The foregoing process makes it possible to determine as the position at which the information tag is to be introduced the position which is considered to have the least effect on the living organism.

A detailed description is given below, with reference to the flowchart shown in FIG. 38, of the process for reading out information from the information tag shown in FIG. 9.

The buffer sequence eliminating unit 203 of the information acquiring apparatus 33 receives the base sequence 457 from the sequencer 32. In Step S901 shown in FIG. 38, it removes the buffer sequence (spacer) from the base sequence 457. In other words, as shown in FIG. 28, the base sequence 457 has the buffer sequence (spacer) inserted at intervals of k bases. Thus, the buffer sequence eliminating unit 203 eliminates this buffer sequence and supplies the base sequence data (having the remaining base sequence connected thereto) to the error correcting unit 204.

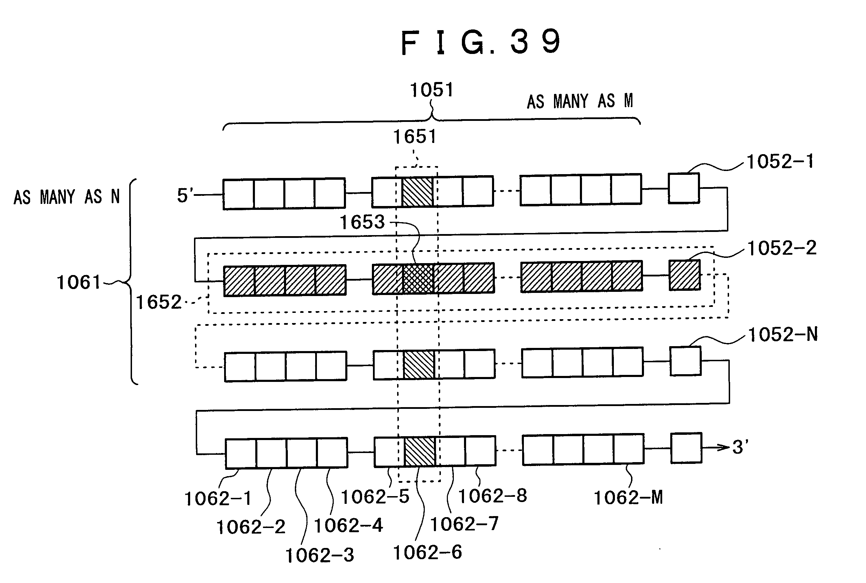

In Step S902, the error correcting unit 204 executes the process for error correction. An example of error is shown in FIG. 39. In the case of the example shown in FIG. 39, errors are found in the vertical checksum 1651 and in the horizontal checksum 1652. The base 1653 at their intersection is an error.

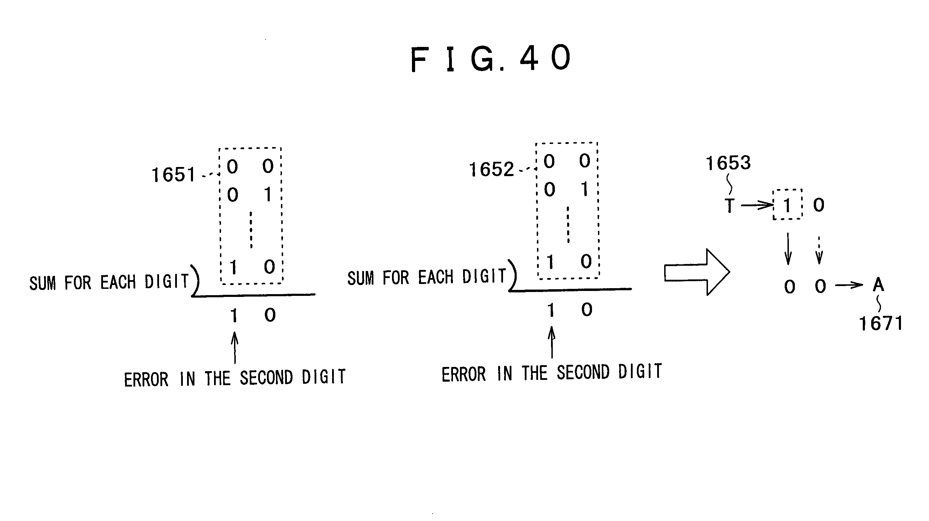

FIG. 40 shows how to correct the base information in the case where the error is at the second order. If all bits representing the bases of the vertical checksum 1651 and the horizontal checksum 1652 are summed up for each order, the sum at the order 1 should be 0 if there are no errors. However, in FIG. 40, the second order is 1. This suggests that the second order is an error in the base 1653 having an error. Therefore, it is possible to obtain the correct base species 1671 by inverting the second order of the binary number corresponding to the base 1652.

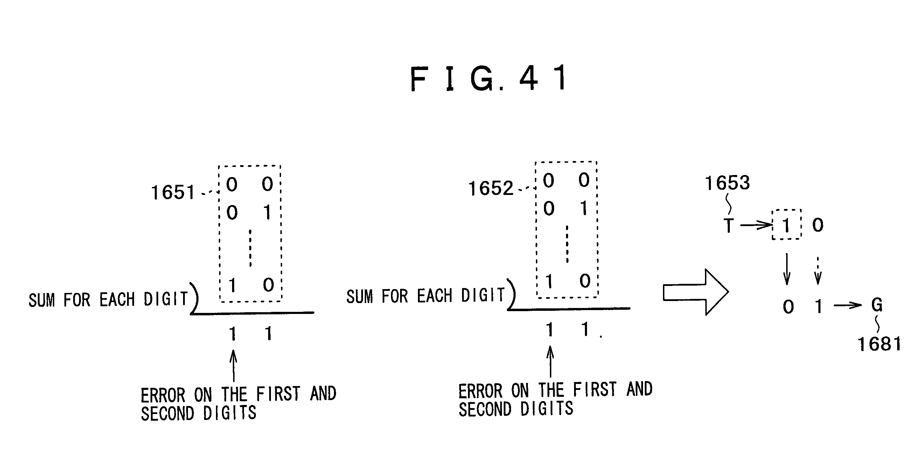

FIG. 41 shows how to correct the base information when there is an error in the first and second orders. Assuming that all bits representing the bases of the vertical checksum 1651 and the horizontal checksum 1652 are summed up for each order, the sum at the order 1 is such that the first order and the second order are 1. This suggests that the first and second orders are errors in the base 1653 having an error. Therefore, it is possible to obtain the correct base species 1681 by inverting the first and second orders of the binary number corresponding to the base 1652.

After the error correction process has been executed as mentioned above, the error correcting unit 204 supplies the error-corrected base sequence data to the sequence converting unit 205. Then, the process proceeds to Step S903.

In Step S903, the sequence converting unit 205 converts the base sequence data supplied from the error correcting unit 204 into binary data according to the correspondence relation shown in FIG. 22, and then it supplies the binary data to the data expanding unit 206. Incidentally, this binary data is the same data as the compressed data generated by the data compressing unit 105 of the sequence designing apparatus 12.

In Step S904, the data expanding unit 206 receives the binary data (compressed data) from the sequence converting unit 205 and then it expands the compressed data according to the decoding system corresponding to the compression coding system which has been executed by the data compressing unit 105.

In Step S905, the data expanding unit 206 displays the information for introduction on the display unit 207 according to the data which has been expanded in Step S904.

The process for reading information from the information tag is executed as mentioned above.

In the meantime, the foregoing description is based on the assumption that the information tag is introduced into the intron region (complementary sequence of the untranslated region) of the gene coded with protein. However, it is possible to insert the information tag into any other part than the gene coded with protein.

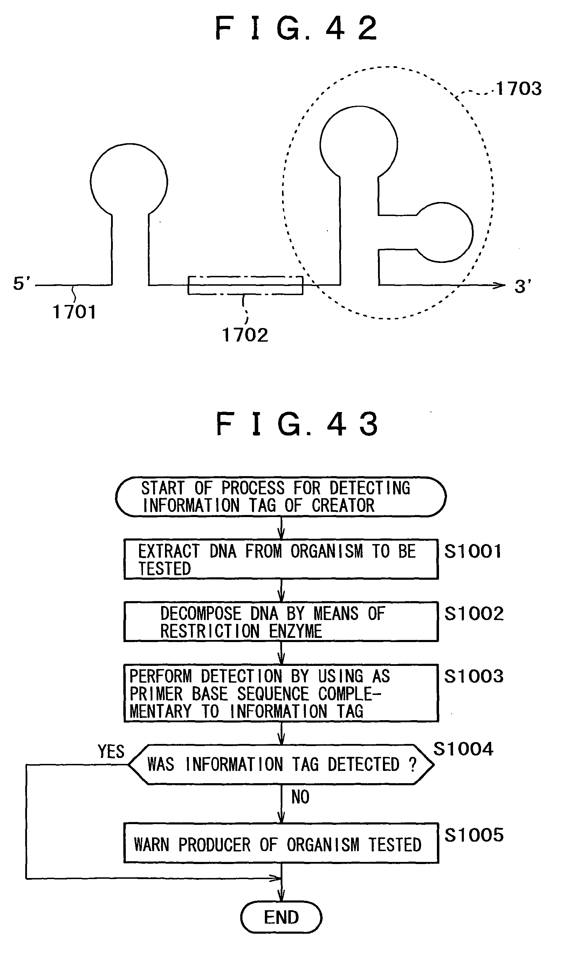

FIG. 42 shows an example of DNA 1701 which is a part other than the gene coded with protein. The gene not coded with protein controls the expression of ribosome RNA and other genes. In view of the fact that RNA has a folded three-dimensional structure so as to perform its function, it is considered that the secondary structure site 1703 relating to the tertiary structure and the evolutionarily preserved base sequence have some functions or other. Consequently, it is necessary to find out the base sequence 1702 other than that and insert the information into it. Incidentally, the base sequence 1702 may be determined in the same way as that of the process for determining the introduction determining process shown in FIG. 36.

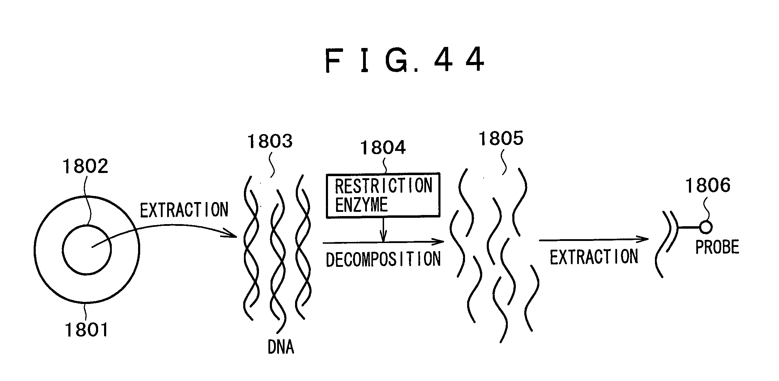

By the way, the creator 11 of the maker 2 previously knows the base sequence of the information tag. Therefore, it would be more convenient if one can know the information tag introduced into the organism 14 without taking trouble to execute the process shown in FIG. 9. Also, it would be possible that a maker other than the maker 2 commercializes an organism having a similar information tag introduced thereinto. Therefore, it is desirable that the maker 2 can simply check to see if other makers commercialize similar products. So, a description is given below, with reference to the flowchart shown in FIG. 43 in conjunction with FIG. 44, of the process for detecting the information tag of the creator 11.

In Step S1001 shown in FIG. 43, the creator 11 extracts the DNA 1803 from the nucleus 1802 of the cell 1801 of the organism to be tested, as shown in FIG. 44. In Step S1002, the creator 11 decomposes the DNA into DNA fragments 1805 by using the restriction enzyme 1804 which recognizes the base sequence at the end of the base sequence introduced, as shown in FIG. 44. The creator 11 fixes the DNA fragments 1805 on a nylon film or the like. In Step S1003, the creator 11 adds the probe 1806 (with a fluorescent substance binding to the base having the base sequence complementary to the information tag) to the container holding the DNA fragments 1805. This step hybridizes the probe 1806 with the DNA fragments (out of the DNA fragments 1805) which are coded with the information tag. After that, the probe which has not been hybridized is washed away.

In Step S1004, the creator 11 irradiates the nylon film with laser beams. Irradiation with laser beams causes the fluorescent substance to develop a color if a hybrid is formed by the probe 1806 and the DNA fragments fixed on the nylon film. In this way it is possible to determine whether or not the desired information tag is present in the cell 1801. If the creator 11 determines that the information tag is present in the cell 1801, then the process proceeds to Step S1005. In Step S1005, the creator 11 issues a warning to the maker who supplies the organism 1801 of interest.

In the way mentioned above, the creator 11 detects the information tag.

According to the present invention as explained above, it is possible to introduce information into a living organism without fail, while minimizing the effect on a living organism.

Incidentally, the forgoing process executed by the creator 11 or the user 31 may be carried out entirely or partly by an industrial robot.

The loxP used in the process explained above may be replaced by any other base sequence having the same function as the loxP.

The error-correcting system explained above may be replaced by any other one.

The above-mentioned processes may be accomplished by means of either hardware or software. In the case that the processes are executed by software, following computers are utilized: one which originally including a hardware exclusive for the program constructing software to execute above-mentioned processes, and one which can execute various functions by installing various programs, for example, general purpose personal computer. In this case the later one accomplishes the above-mentioned processes by installing the program which executes the serial processes from a recording medium or the like.

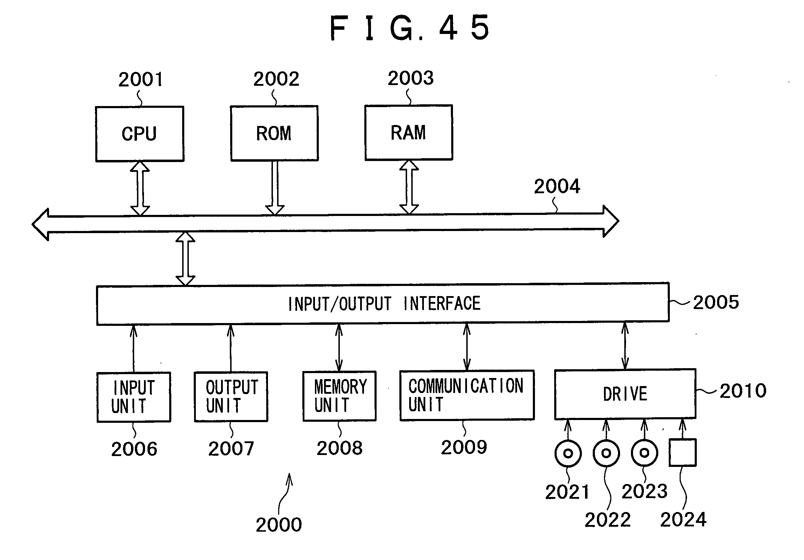

FIG. 45 shows an example of the internal structure of the personal computer 2000 designed to execute the above-mentioned processes. The personal computer has the CPU (Central Processing Unit) 2001, which executes various processes according to the program stored in the ROM (Read Only Memory) 2002. The RAM (Random Access Memory) 2003 stores data and programs necessary for the CPU 2001 to executes various processes. To the input/output interface 2005 is connected the input unit 2006 consisting of a mouse, keyboard, microphone, AD converter, etc., so that signals entered into the input unit 2006 are output to the CPU 2001. To the input/output interface 2005 is also connected the output unit 2007 including a display, speaker, DA converter, etc.

Moreover, the input/output interface 2005 is connected to a memory unit 2008 comprised of a hard disk etc., and a communication unit 2009 communicating data with the other apparatus via a network such as the Internet. The drive 2010 reads and writes data from and to such recording media as magnetic disc 2021, optical disc 2022, magneto-optical disc 2023, and semiconductor memory 2034.

The medium to store the executable programs installed in the computer includes, as shown in FIG. 45, magnetic disc 2021 (including flexible disc), optical disc (including CD-ROM (Compact Disc-Read Only Memory) and DVD (Digital Versatile Disc)), package media such as semiconductor memory 2024, ROM 2002 to store programs temporarily or permanently, and hard disc constituting the memory unit 2008. The storing of programs in the program storage media may be accomplished through an interface such as router and modem by means of wire or wireless communication media such as local area network, Internet, and digital satellite broadcasting.

In this specification, the steps describing the programs stored in the recoding medium may be carried out sequentially in the chronological order as listed or parallel or individually.

In this specification, the system denotes the apparatus as a whole consisting of a plurality of apparatus.

The present invention is not limited to the details of the above described preferred embodiments. The scope of the invention is defined by the appended claims and all changes and modifications as fall within the equivalence of the scope of the claims are therefore to be embraced by the invention.

Various respective aspects and features of the invention are defined in the appended claims. Features from the dependent claims may be combined with features of the independent claims as appropriate and not merely as explicitly set out in the claims.

## Claims

1. A DNA to be introduced into a biogenic gene which is encoded with information to be introduced into a biogenic DNA of a genetically manipulated organism and which has an error-correcting base inserted thereinto.

2. The DNA to be introduced into a biogenic gene as defined in Claim 1, which additionally has buffer bases so that it decreases in homology to the base sequence of DNA of more than one species of organism.

3. The DNA to be introduced into a biogenic gene as defined in Claim 1, which additionally has a drug-tolerant gene for positive selection.

4. The DNA to be introduced into a biogenic gene as defined in Claim 1, which additionally has a drug-tolerant gene for negative selection.

5. The DNA to be introduced into a biogenic gene as defined in Claim 1, which additionally has the base of intron sequence which is recognized as the intron region by said genetically manipulated organism.

6. A vector for gene introduction which is **characterized in** containing the DNA as defined in Claim 1.

7. A cell which is **characterized in** possessing the DNA as defined in Claim 1.

8. A method for introduction of information into a biogenic gene, said method comprising steps of:
designing the base sequence of a DNA to be introduced into a biogenic DNA, said DNA being encoded with information to be introduced into a biogenic DNA of a genetically manipulated organism and having an error-correcting base inserted thereinto,
synthesizing said DNA having the thus designed base sequence, and
introducing the thus synthesized DNA into a genetically manipulated gene of said biogenic DNA.

9. The method for introduction of information into a biogenic gene as defined in Claim 8, which is accomplished in such a way that said DNA is introduced into a region not encoded with protein out of the base sequence of the genetically manipulated gene, when said DNA is introduced into said genetically manipulated gene.

10. The method for introduction of information into a biogenic gene as defined in Claim 9, wherein the region not encoded with protein is an intron region.

11. The method for introduction of information into a biogenic gene as defined in Claim 9, wherein the region not encoded with protein is a region of the base sequence complementary to the untranslated region which is not translated into said protein, of the base sequence of mRNA to which said gene has been transcribed, out of said biogenic DNA.

12. The method for introduction of information into a biogenic gene as defined in Claim 11, wherein said base sequence of said DNA to be introduced into said biogenic DNA further contains an intron sequence which is recognized as the intron region by said genetically manipulated organism.

13. The method for introduction of information into a biogenic gene as defined in Claim 9, which method, before introducing said synthesized DNA into said genetically manipulated gene, includes steps of
acquiring a base sequence pattern similar to that present in more than one species of organisms according to the base sequence of DNA of more than one species of organisms, and
selecting a site of base sequence having the least similarity to said base sequence pattern from said region not encoded with protein of said genetically manipulated gene,
said method further includes a step of
introducing said synthesized DNA into said site selected as the site having the least similarity to said base sequence pattern at the time of introducing said DNA into said genetically manipulated gene.

14. The method for introduction of information into a biogenic gene as defined in Claim 8, which includes steps of:
acquiring a base sequence pattern similar to that present in more than one species of organisms according to the base sequence of DNA of more than one species of organisms before designing said base sequence of said DNA to be introduced into said biogenic DNA, and
inserting a buffer base sequence so that the similarity to the acquired base sequence pattern becomes least, thereby designing the said base sequence of said DNA to be introduced into said biogenic DNA at the time of designing said base sequence of said DNA to be introduced into biogenic DNA.

15. A data processing apparatus which comprises:
converting means to convert binary data containing information to be introduced into a biogenic DNA of a genetically manipulated organism into base sequence data and
inserting means to insert base data for error correction into the base sequence data obtained by said converting means.

16. A data processing method which comprises steps of:
converting binary data containing information to be introduced into a biogenic DNA of a genetically manipulated organism into base sequence data, and
inserting base data for error correction into the base sequence data obtained by said converting step.

17. A recording medium storing a computer-readable program including steps of:
converting binary data containing information to be introduced into a biogenic DNA of a genetically manipulated organism into base sequence data, and
inserting base data for error correction into the base sequence data obtained by said converting step.

18. A program which causes a computer to execute steps of:
converting binary data containing information to be introduced into a biogenic DNA of a genetically manipulated organism into base sequence data, and
inserting base data for error correction into the base sequence data obtained by said converting step.

19. A data processing apparatus which comprises:
correcting means to correct errors in base sequence data according to the base sequence for error correction inserted into the base sequence data, and
converting means to convert the base sequence data, which has been error-corrected by the correcting means, into binary data.

20. A data processing method which comprises steps of:
correcting errors in base sequence data according to the base sequence for error correction inserted into the base sequence data, and
converting the base sequence data, which has been error-corrected by the correcting step, into binary data.

21. A recording medium storing a computer-readable program including steps of:
correcting errors in base sequence data according to the base sequence for error correction inserted into the base sequence data, and
converting the base sequence data, which has been error-corrected by the correcting step, into binary data.

22. A program which causes a computer to execute steps of:
correcting errors in base sequence data according to the base sequence for error correction inserted into the base sequence data, and
converting the base sequence data, which has been error-corrected by the correcting step, into binary data.
